(19) **Europäisches Patentamt / European Patent Office / Office européen des brevets**

(11) **EP 4 765 156 A1**

(12) **EUROPEAN PATENT APPLICATION**
published in accordance with Art. 153(4) EPC

(43) Date of publication:
**24.06.2026  Bulletin 2026/26**

(21) Application number: **24911575.9**

(22) Date of filing: **28.12.2024**

(51) International Patent Classification (IPC):
**G16H 50/30** (2018.01)     **G16H 20/60** (2018.01)
**A61B 5/145** (2006.01)     **A61B 5/024** (2006.01)
**A61B 5/00** (2006.01)

(52) Cooperative Patent Classification (CPC):
**A61B 5/00; A61B 5/024; A61B 5/145; G16H 10/60;
G16H 20/60; G16H 50/30; G16H 50/70**

(86) International application number:
**PCT/CN2024/143474**

(87) International publication number:
**WO 2025/140661 (03.07.2025 Gazette 2025/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.12.2023  CN 202311870197**

(71) Applicant: **HUAWEI TECHNOLOGIES CO., LTD.
Shenzhen 518129 (CN)**

(72) Inventors:
• **TIAN, Kan**
  **Shenzhen, Guangdong 518129 (CN)**
• **PAN, Jun**
  **Shenzhen, Guangdong 518129 (CN)**
• **XIE, Songlin**
  **Shenzhen, Guangdong 518129 (CN)**
• **ZHANG, Jie**
  **Shenzhen, Guangdong 518129 (CN)**

(74) Representative: **Maiwald GmbH
Engineering
Elisenhof
Elisenstrasse 3
80335 München (DE)**

(54) **BLOOD GLUCOSE MANAGEMENT METHOD, USER INTERFACE, AND RELATED APPARATUS**

(57)     A blood glucose management method, a user interface, and a related apparatus are provided. According to the blood glucose management method, a first meal time can be determined based on historical blood glucose values of a user; a second meal time can be determined based on one or more of the following: a meal time input by the user, a hand movement of the user, and a heart rate of the user; and a third meal time can be determined based on the first meal time and the second meal time, where the third meal time is used to evaluate a blood glucose health status of the user. A meal time of the user is predicted based on historical meal times of the user that are determined in a plurality of manners, so that an electronic device can evaluate a blood glucose status of the user more accurately.

Determine a historical meal time t1 of a user based on historical blood glucose values of the user — S201

Determine a historical meal time t2 based on any one or more of the following: a historical meal time input by the user, a hand movement of the user, and a heart rate of the user — S202

Determine a meal time t0 of the user based on the historical meal time t1 and the historical meal time t2 — S203

When the meal time t0 of the user is reached, output prompt information, where the prompt information is used to prompt the user that a predicted meal time has been reached currently — S204

FIG. 4

EP 4 765 156 A1

## Description

This application claims priority to Chinese Patent Application No. 202311870197.X, filed with the China National Intellectual Property Administration on December 29, 2023 and entitled "BLOOD GLUCOSE MANAGEMENT METHOD, USER INTERFACE, AND RELATED APPARATUS", which is incorporated herein by reference in its entirety.

## TECHNICAL FIELD

This application relates to the terminal field, and in particular, to a blood glucose management method, a user interface, and a related apparatus.

## BACKGROUND

In recent years, the incidence of diabetes has been gradually increasing. Diabetes is a long-term chronic disease. If blood glucose in a human body is not controlled in a timely manner, various tissues, especially eyes, kidneys, a heart, blood vessels, and nerves, are prone to chronic damage and functional impairment. Daily behavior and self-management of a user are key factors that affect a control status of diabetes.

## SUMMARY

This application provides a blood glucose management method, a user interface, and a related apparatus, to accurately calculate a meal time of a user.

According to a first aspect, an embodiment of this application provides a blood glucose management method. The method is applied to an electronic device. The method includes: determining a first meal time based on historical blood glucose values of a user; determining a second meal time based on any one or more of the following: a meal time input by the user, a hand movement of the user, and a heart rate of the user; and determining a third meal time based on the first meal time and the second meal time, where the third meal time is used by the electronic device to evaluate a blood glucose status of the user.

According to the method provided in the first aspect, a meal pattern of the user in daily diet may be summarized with reference to historical meal times estimated by the user in a plurality of manners. In this way, a future meal time of the user is accurately predicted, so that the electronic device provides a more accurate blood glucose evaluation result based on the meal time.

With reference to the first aspect, in an implementation, after the electronic device determines the third meal time based on the first meal time and the second meal time, the method further includes: outputting first prompt information when the third meal time is reached, where the first prompt information indicates that a current time reaches a meal time predicted by the electronic device.

In this way, based on the first prompt information, the user may learn that the meal time predicted by the electronic device has been reached currently.

With reference to the first aspect, in an implementation, the method further includes: detecting a first operation performed on the first prompt information, and determining the third meal time as a meal time of a first dietary event of the user, or determining a fourth meal time as a meal time of a first dietary event of the user, where the fourth meal time is different from the third meal time.

This is because the meal time predicted by the electronic device may not be an actual meal time of the user. Therefore, after the predicted meal time is reached, the electronic device may remind the user to confirm or modify the time, so that the electronic device can provide a more accurate blood glucose evaluation result based on the actual meal time of the user.

With reference to the first aspect, in an implementation, the fourth meal time is a time input by the user or a time determined based on the third meal time.

In other words, if the time predicted by the electronic device is not an actual meal time of the user, the user may manually input the actual meal time, or the electronic device determines a calibrated meal time.

With reference to the first aspect, in an implementation, after determining the fourth meal time as the meal time of the first dietary event of the user and detecting the first operation performed on the first prompt information, the method further includes: displaying the first prompt information after a first time interval; and detecting a second operation performed on the first prompt information, and determining the current time as the fourth meal time.

In other words, if the time predicted by the electronic device is not the actual meal time of the user, the electronic device may remind the user to determine the meal time after a time period until the user determines the meal time.

With reference to the first aspect, in an implementation, before or after determining the meal time of the first dietary event, the method further includes: determining a first food to be ingested by the user and a first intake of the first food in the first dietary event; determining a first blood glucose curve based on information about the first food, the first intake of the first food, and a blood glucose value of the user before a current time point, where the first blood glucose curve is a blood glucose change of the user, predicted by the electronic device, under influence of the first dietary event; and displaying the first blood glucose curve.

In this way, before the user has a meal, the user may view a blood glucose change that is predicted by the electronic device and that may occur after the user has the meal, so that the user can adjust, in a timely manner, the food to be ingested and the intake of the food in this dietary event, and that the user can take a timely measure before the dietary intake is to endanger physical health of the user, to ensure the physical health of the user.

With reference to the first aspect, in an implementation, the method further includes: determining a second blood glucose curve based on the blood glucose value of the user before the current time point, where the second blood glucose curve is a blood glucose change of the user, predicted by the electronic device, in absence of the first dietary event; and displaying the second blood glucose curve.

In this way, the user may compare a future blood glucose change of the user under the influence of the dietary event with a future blood glucose change of the user in absence of the dietary event, to highlight an association between the dietary event and the blood glucose of the user, so that the user better plans a healthy dietary habit, thereby effectively avoiding risks caused by hyperglycemia and hypoglycemia.

With reference to the first aspect, in an implementation, determining the first blood glucose curve based on the information about the first food, the first intake of the first food, and the blood glucose value of the user before the current time point specifically includes: determining the first blood glucose curve based on an absorption index of the first food for the user, the first intake of the first food, and the blood glucose value of the user before the current time point, where the absorption index of the first food for the user is related to the first food and a physical condition of the user.

In other words, the electronic device may predict a future blood glucose status of the user based on an absorption index of a food for the user, to provide an accurate blood glucose prediction result for the user.

With reference to the first aspect, in an implementation, the method further includes: determining the absorption index of the first food for the user based on a blood glucose value within first duration and the physical condition after the user ingests a second intake of the first food, where the physical condition includes any one or more of the following: an age, a height, a weight, a disease type, and medication information.

It can be learned that, in this application, considering that different people have different food absorption conditions, a customized food absorption index applicable to an individual is obtained through calculation with reference to the physical condition of the user.

With reference to the first aspect, in an implementation, the electronic device stores absorption indexes of a plurality of foods for the user.

In other words, the electronic device may obtain absorption indexes of a plurality of foods through measurement and save the absorption indexes locally. In this way, based on absorption indexes of various foods, the user can learn about a degree of influence of each food on blood glucose of the user, so that the user can arrange diet more appropriately.

With reference to the first aspect, in an implementation, the method further includes: determining a third intake of the user based on a blood glucose value of the user within second duration after the third meal time; and displaying the third intake.

In this way, the user can learn about the intake of each meal, and control the intake of each meal, so that the user can develop a regular dietary habit and control a stable fluctuation of blood glucose.

With reference to the first aspect, in an implementation, the method further includes: obtaining a blood glucose value of the user within third duration after the third meal time; and displaying a third blood glucose curve, where the third blood glucose curve is determined based on a plurality of blood glucose values of the user within the third duration after the third meal time.

In this way, after the user has the meal, the user can learn about an actual blood glucose status of the user under influence of the dietary event. This helps the user learn about influence of diet on blood glucose more intuitively, so that the user can control blood glucose based on the diet.

With reference to the first aspect, in an implementation, determining the first meal time based on the historical blood glucose values of the user specifically includes: determining the first meal time based on a rate of increase in the historical blood glucose values of the user.

Because blood glucose tends to increase after the user has the meal, the meal time of the user may be accurately determined based on the rate of increase in the blood glucose values.

According to a second aspect, an embodiment of this application provides a blood glucose management method. The method is applied to an electronic device. The method includes: obtaining a blood glucose value within first duration after a user ingests a second intake of a first food; and determining an absorption index of the first food for the user based on the blood glucose value within the first duration and a physical condition of the user.

According to the method provided in the second aspect, a customized food absorption index applicable to an individual may be obtained through calculation with reference to the physical condition of the user, so that the user can formulate a better dietary plan based on an actual physical condition of the user.

With reference to the second aspect, in an implementation, the physical condition includes any one or more of the following: an age, a height, a weight, a disease type, and medication information.

With reference to the second aspect, in an implementation, the method further includes: determining the first food to be ingested by the user and a first intake of the first food in a first dietary event; determining a first blood glucose curve based on the absorption index of the first food for the user, the first intake of the first food, and a blood glucose value of the user before a current time point, where the first blood glucose curve is a blood glucose change of the user, predicted by the electronic device, under influence of the first dietary event; and displaying the first blood glucose curve.

With reference to the second aspect, in an implementation, the method further includes: determining a second blood glucose curve based on the blood glucose value of the user before the current time point, where the second blood glucose curve is a blood glucose change of the user, predicted by the electronic device, in absence of the first dietary event; and displaying the second blood glucose curve.

With reference to the second aspect, in an implementation, the method further includes: determining a first meal time based on historical blood glucose values of the user; determining a second meal time based on any one or more of the following: a meal time input by the user, a hand movement of the user, and a heart rate of the user; and determining a third meal time based on the first meal time and the second meal time, where determining the first food to be ingested by the user and the first intake of the first food in the first dietary event specifically includes: determining the first food to be ingested by the user and the first intake of the first food in the first dietary event after the third meal time is reached.

With reference to the second aspect, in an implementation, determining, by the electronic device, the first meal time based on the historical blood glucose values of the user specifically includes: determining the first meal time based on a rate of increase in the historical blood glucose values of the user.

With reference to the second aspect, in an implementation, the method further includes: outputting first prompt information when the third meal time is reached, where the first prompt information indicates that a current time reaches a meal time predicted by the electronic device.

With reference to the second aspect, in an implementation, after outputting the first prompt information, the method further includes: detecting a first operation performed on the first prompt information, and determining the third meal time as a meal time of the first dietary event, or determining a fourth meal time as a meal time of the first dietary event, where the fourth meal time is different from the third meal time.

With reference to the second aspect, in an implementation, the fourth meal time is a time input by the user or a time determined based on the third meal time.

With reference to the second aspect, in an implementation, after determining the fourth meal time as the meal time of the first dietary event of the user and detecting the first operation performed on the first prompt information, the method further includes: displaying the first prompt information after a first time interval; and detecting a second operation performed on the first prompt information, and determining the current time as the fourth meal time.

With reference to the second aspect, in an implementation, the method further includes: determining a third intake of the user based on a blood glucose value of the user within second duration after the third meal time; and displaying the third intake.

With reference to the second aspect, in an implementation, the method further includes: obtaining a blood glucose value of the user within third duration after the third meal time; and displaying a third blood glucose curve, where the third blood glucose curve is determined based on a plurality of blood glucose values of the user within the third duration after the third meal time.

With reference to the second aspect, in an implementation, the electronic device stores absorption indexes of a plurality of foods for the user.

According to a third aspect, an embodiment of this application provides an electronic device, including a memory, one or more processors, and one or more programs. When the one or more processors execute the one or more programs, the electronic device is enabled to implement the method according to any one of the first aspect or the implementations of the first aspect, or the method according to any one of the second aspect or the implementations of the second aspect.

According to a fourth aspect, an embodiment of this application provides a computer-readable storage medium, including instructions. When the instructions are run on an electronic device, the electronic device is enabled to perform the method according to any one of the first aspect or the implementations of the first aspect, or the method according to any one of the second aspect or the implementations of the second aspect.

According to a fifth aspect, an embodiment of this application provides a computer program product. When the computer program product is run on a computer, the computer is enabled to perform the method according to any one of the first aspect or the implementations of the first aspect, or the method according to any one of the second aspect or the implementations of the second aspect.

For descriptions of beneficial effects in the second aspect to the fifth aspect, refer to descriptions of beneficial effects in the first aspect.

## BRIEF DESCRIPTION OF DRAWINGS

FIG. 1 is a diagram of a structure of a communication system 1000 according to an embodiment of this application;

FIG. 2 is an overall schematic flowchart of a blood glucose management method according to an embodiment of this application;

FIG. 3A and FIG. 3B show page content displayed by an electronic device 100 and used to evaluate a blood glucose health status of a user according to an embodiment of this application;

FIG. 4 is a schematic flowchart for determining a meal time of a user according to an embodiment of this application;

FIG. 5A to FIG. 5D show some user interfaces displayed by an electronic device 100 after a meal time t0 of a user is reached according to an embodiment of this application;

FIG. 6A to FIG. 6F show some user interfaces used in a process of measuring an absorption index of a food by an electronic device 100 according to an embodiment of this application;

FIG. 7 is a schematic flowchart of another blood glucose management method according to an embodiment of this application;

FIG. 8 is a diagram of a hardware structure of an electronic device 100 according to an embodiment of this application;

FIG. 9 is a block diagram of a software structure of an electronic device 100 according to an embodiment of this application; and

FIG. 10 is a diagram of a structure of a blood glucose management apparatus 300 according to an embodiment of this application.

## DESCRIPTION OF EMBODIMENTS

The following clearly describes technical solutions in embodiments of this application in detail with reference to accompanying drawings. In the descriptions of embodiments of this application, unless otherwise specified, "/" indicates "or". For example, the term "A/B" may indicate A or B. The term "and/or" in this specification describes only an association relationship between associated objects, and indicates that three relationships may exist. For example, "A and/or B" may indicate the following three cases: Only A exists, both A and B exist, and only B exists. In addition, in the descriptions of embodiments of this application, the term "a plurality of" indicates two or more.

Hereinafter, the terms "first" and "second" are merely intended for a purpose of description, and shall not be understood as an indication or implication of relative importance or an implicit indication of a quantity of indicated technical features. Therefore, a feature defined by "first" or "second" may explicitly or implicitly include one or more features. In the descriptions of embodiments of this application, unless otherwise specified, the term "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is source code written in a specific computer language, for example, Java or an extensible markup language (extensible markup language, XML). Interface source code is parsed and rendered on an electronic device, and is finally presented as content that can be identified by the user. The user interface is usually represented in a form of a graphical user interface (graphical user interface, GUI), and is a user interface related to a computer operation and displayed in a graphical manner. The user interface may be a visual interface element, for example, a text, an icon, a button, a menu, a tab, a text box, a dialog box, a status bar, a navigation bar, or a widget displayed on a display of the electronic device.

In daily behavior of a user, diet is one of key factors influencing blood glucose fluctuations. By paying attention to a meal time of the user and using the meal time as a starting point, a blood glucose status of the user under the influence of the diet can be evaluated with reference to blood glucose of the user. This enables the user to control the blood glucose of the user in a timely manner from a perspective of diet, so that the blood glucose of the user can fluctuate within a normal range, thereby ensuring physical health of the user.

An embodiment of this application provides a blood glucose management method. According to the method, blood glucose values historically collected by a continuous blood glucose monitoring (continuous glucose monitoring, CGM) device may be obtained, a historical meal time t1 of a user is calculated by using the blood glucose values, and a historical meal time t2 of the user is determined based on any one or more of the following: a historical meal time input by the user, a hand movement of the user, and a heart rate of the user; and then a meal time t0 of the user may be determined based on the historical meal time t1 and the historical meal time t2, and an electronic device 100 may evaluate a blood glucose status of the user based on the meal time t0 of the user.

The CGM device may subcutaneously implant a biosensor (microneedle sensor) in contact with interstitial fluid, to determine a glucose concentration in the interstitial fluid, and then determine a blood glucose value of the user based on the glucose concentration in the interstitial fluid.

After the user eats, there is generally a change process in which the blood glucose gradually increases. Therefore, the historical meal time of the user may be calculated based on the historical blood glucose change process of the user.

In addition, the user may also manually input the historical meal time based on memory. Alternatively, because a hand movement is usually involved in a meal process of the user, or a heart rate may change in a meal process of the user, if the electronic device 100 is a wearable device such as a watch or a band, or the electronic device 100 is connected to a wearable device such as a watch or a band, the electronic device 100 may determine, based on a hand movement, a heart rate value, and the like that are detected by the wearable device, whether the user has a meal, and record a meal time of the user when determining that the user has a meal.

It can be learned that, in this embodiment of this application, a meal pattern of the user in daily diet may be summarized with reference to historical meal times of the user that are estimated in a plurality of manners, so that the meal time of the user is accurately predicted. This enables the electronic device 100 to provide a more accurate blood glucose evaluation result, an opinion and a suggestion, or the like based on the meal time.

Dietary therapy is one of the most fundamental and important treatment methods for diabetes. Appropriate diet can effectively control diabetes. Many official organizations recommend that people, and especially diabetics, should appropriately select foods and control diet with reference to a food glycemic index (glycemic index, GI) table, and suggest that total carbohydrate content and GI values of foods should be indicated on food labels.

A GI is an indicator used to measure a rate and a capability of blood glucose increase after a user consumes a food. A higher GI indicates a stronger postprandial blood glucose response caused by the food. Foods with low GIs are more suitable for diabetics, and foods with high GIs are not suitable for diabetics. Based on GIs of foods, the user may select foods that are more suitable for physical health of the user, and control the blood glucose of the user to fluctuate within a normal range.

However, existing GI values of foods are all based on this assumption: A same food has a same blood glucose effect on different individuals. Therefore, it is inappropriate for different users to control their blood glucose merely based on unified GI values of foods. The GI values do not take into account individual differences such as food absorption capabilities of different users and food absorption conditions of diabetics under influence of medicine.

An embodiment of this application further provides a blood glucose management method. According to the method, a blood glucose value of a user in a time period after the user ingests a second intake of a first food may be obtained, and an absorption index of the first food for the user is determined based on the blood glucose value and a physical condition of the user.

The physical condition may include one or more of the following: an age, a height, a weight, a disease type, medication information, and the like.

Currently known GI values of foods are reference values suitable for a general population and obtained through measurement based on a healthy population. However, in this application, considering that different people have different food absorption conditions, a customized food absorption index applicable to an individual is obtained through calculation with reference to the physical condition of the user, so that the user formulates a better dietary plan based on an actual physical condition of the user.

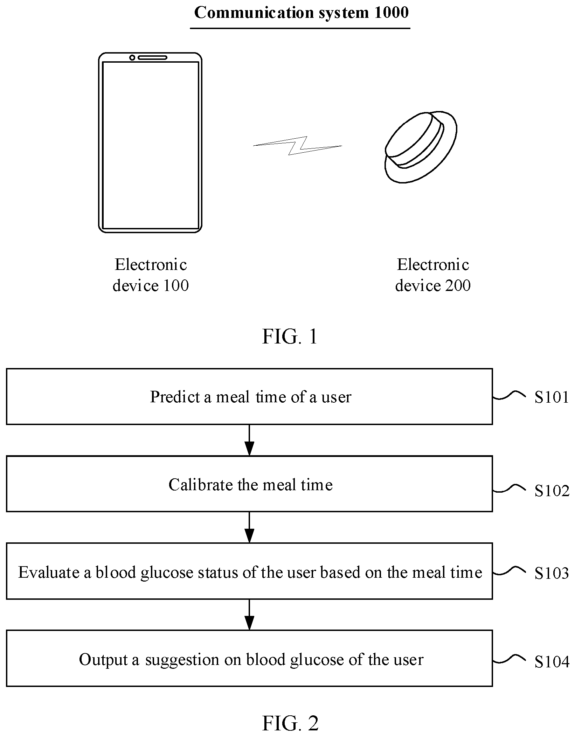

**FIG. 1 is a diagram of a structure of a communication system 1000 according to an embodiment of this application.**

As shown in FIG. 1, the communication system 1000 may include an electronic device 100 and an electronic device 200.

The electronic device 100 may be a mobile phone, a computer, a wearable device, or electronic devices of other types. The electronic device 100 may obtain a blood glucose value collected by the electronic device 200, determine a historical meal time t1 of a user based on the blood glucose value, determine a historical meal time t2, where the historical meal time t2 may be determined based on any one or more of the following: a meal time manually input by the user, a hand movement of the user, and a heart rate of the user, determine a meal time t0 of the user based on the historical meal time t1 and the historical meal time t2, and evaluate a blood glucose status or the like of the user based on the meal time t0 of the user. In addition, the electronic device 100 may further obtain, by using the electronic device 200, a blood glucose value after the user ingests a food, and determine an absorption index of the food for the user based on the blood glucose value and a physical condition of the user.

The electronic device 200 may be a CGM device. The electronic device 200 may be worn on the user, and is configured to collect the blood glucose value of the user, and send the blood glucose value of the user to the electronic device 100.

A communication connection may be established between the electronic device 100 and the electronic device 200. Specifically, the communication connection may be a wired connection, or may be a wireless connection. The wireless connection may be a short-distance connection like a high-fidelity wireless communication (wireless fidelity, Wi-Fi) connection, a Bluetooth connection, an infrared connection, an NFC connection, or a ZigBee connection, or may be a long-distance connection. The long-distance connection includes but is not limited to a long-distance connection based on 2G, 3G, 4G, 5G, and mobile networks of later standard protocols. For example, the electronic device 100 may establish a communication connection to the electronic device 200 through Bluetooth.

In some implementations, considering that the electronic device 200 is worn on the user and can be in contact with the skin of the user, if the electronic device 100 can also be in contact with the skin of the user, the electronic device 100 may communicate with the electronic device 200 through the human skin. For example, the electronic device 100 may obtain, through the human skin, the blood glucose value sent by the electronic device 200. For example, the electronic device 100 and the electronic device 200 may include electrodes. The electronic device 100 and the electronic device 200 may communicate through the human skin by using the electrodes. For example, the electronic device 100 may receive, by using the electrodes, the blood glucose value sent by the electronic device 200.

It may be understood that the communication system 1000 may further include more or fewer electronic devices. For example, if the electronic device 100 is a mobile phone, the communication system 1000 may further include a wearable device such as a watch or a band. The device may determine, based on factors such as a hand movement and/or a heart rate of the user, whether the user has a meal, record a meal time of the user, and send the meal time to the electronic device 100. A quantity of electronic devices included in the communication system 1000 is not limited in this embodiment of this application.

**FIG. 2 is an overall schematic flowchart of a blood glucose management method according to an embodiment of this application.**

As shown in FIG. 2, the blood glucose management method may generally include the following steps.

**S101:** An electronic device 100 predicts a meal time of a user.

The electronic device 100 may summarize a daily dietary pattern of the user based on a historical meal time determined in a historical dietary event of the user, to infer a future meal time of the user.

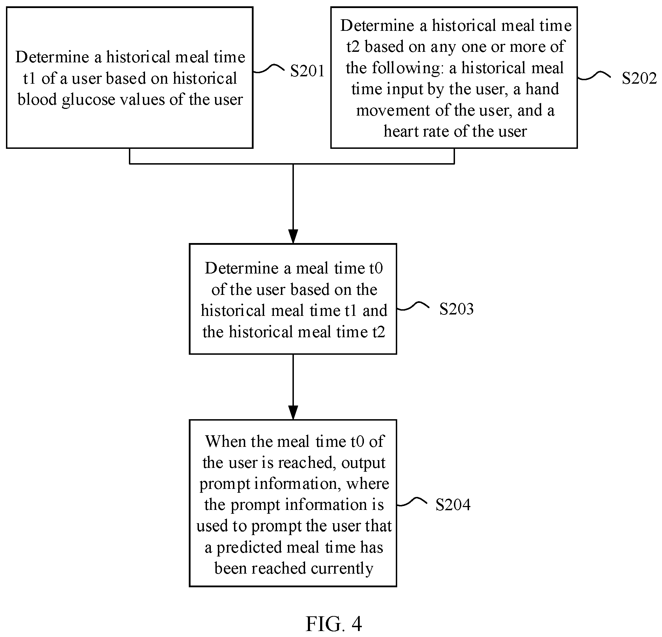

For example, the electronic device 100 may predict the meal time of the user with reference to factors such as historical blood glucose values of the user, a manual input of the user, and/or a hand movement of the user and/or a heart rate of the user. For detailed descriptions about predicting, by the electronic device 100, the meal time of the user, refer to related content in FIG. 4 subsequently. Details are not described herein.

**S102:** The electronic device 100 calibrates the meal time.

After the electronic device 100 predicts the meal time, the meal time may be displayed to the user, and the user calibrates the meal time.

For example, when the predicted meal time is reached, the electronic device 100 may output prompt information to prompt the user whether to start a meal currently, and the user may calibrate the meal time based on the prompt information.

It may be understood that step S102 is an optional step. Alternatively, after the electronic device 100 predicts the meal time of the user, the meal time may not need to be calibrated.

**S103:** The electronic device 100 evaluates a blood glucose status of the user based on the meal time.

After the electronic device 100 obtains the meal time, the electronic device 100 may record, by using the meal time as a starting point, a dietary intake and a blood glucose change of the user after the meal starts, and evaluate, based on the dietary intake, the blood glucose change, and the like, the blood glucose status of the user under influence of a current dietary event.

For example, the electronic device 100 may evaluate the blood glucose health status of the user based on the meal time in the following plurality of manners:

(1) When the meal time is reached, predict a future blood glucose change of the user based on a dietary intake of the dietary event input by the user.

For example, after predicting the meal time, the electronic device 100 may output prompt information when the meal time is reached, to prompt the user to input a dietary amount to be ingested in this dietary event. Before the user has the meal, the electronic device 100 helps the user predict a postprandial blood glucose change in advance, and before the diet adversely affects the blood glucose of the user, the electronic device 100 provides a timely reminder for the user to adjust the diet, to help the user better control the dietary intake.

(2) Calculate an intake of the user in this dietary event based on a blood glucose value of the user in a time period after the meal time and display the intake to the user for viewing.

For example, using the meal time as a starting point, the electronic device 100 may collect statistics on a dietary intake of the user in a time period after the user has the meal, and analyze the dietary intake of the user, so that the user adjusts the dietary intake in a timely manner to control a normal fluctuation of blood glucose.

In some implementations, the electronic device 100 may further organize and collect statistics on intakes in a plurality of dietary events of the user in a time period, to help the user learn about a dietary status of the user from a long-term perspective, and control a stable fluctuation of blood glucose.

(3) Display, to the user, a blood glucose fluctuation of the user starting from the meal time.

Specifically, after obtaining the meal time, the electronic device 100 may obtain, by using the electronic device 200, blood glucose values of the user in a time period after the meal time, and display, to the user, a blood glucose curve generated by connecting the blood glucose values in this time period.

In this way, by using the electronic device 100, the user may learn about a blood glucose change after the user starts the meal, so that the user can control the dietary intake in a timely manner or control, in a timely manner by intervening with an event such as medication or exercise, influence of the dietary event on the blood glucose of the user.

**S104:** The electronic device 100 outputs a suggestion on blood glucose of the user.

In addition, to enable the user to more comprehensively learn about health of the user and make a timely adjustment, in addition to evaluating the blood glucose status of the user based on the meal time, the electronic device 100 may further output the suggestion on blood glucose of the user, for example, recommend an optimal meal time and an optimal intake, and generate a customized diet schedule for the user, to efficiently and intelligently assist the user in completing diet management, so that the user can adjust a dietary habit of the user in a timely manner, or take medicine in a timely manner, strengthen exercise, increase exercise, or the like, thereby reducing occurrences of hyperglycemic or hypoglycemic events, controlling the blood glucose fluctuation of the user, and ensuring physical health of the user.

In some implementations, the electronic device 100 may generate a daily dietary analysis report based on the meal time of the user and blood glucose before and after the meal time of the user. The daily dietary analysis report may include but is not limited to a total daily intake of the user, an intake per meal, a blood glucose curve annotated with the meal time, and the like.

In some other implementations, the electronic device 100 may generate a plurality of dietary analysis reports based on the meal time of the user and blood glucose before and after the meal time of the user. The plurality of dietary analysis reports may include but are not limited to a daily average intake of the user, an intake per meal, an average blood glucose curve annotated with the meal time, a daily per-meal bar chart, statistics of preprandial and postprandial blood glucose data, and the like.

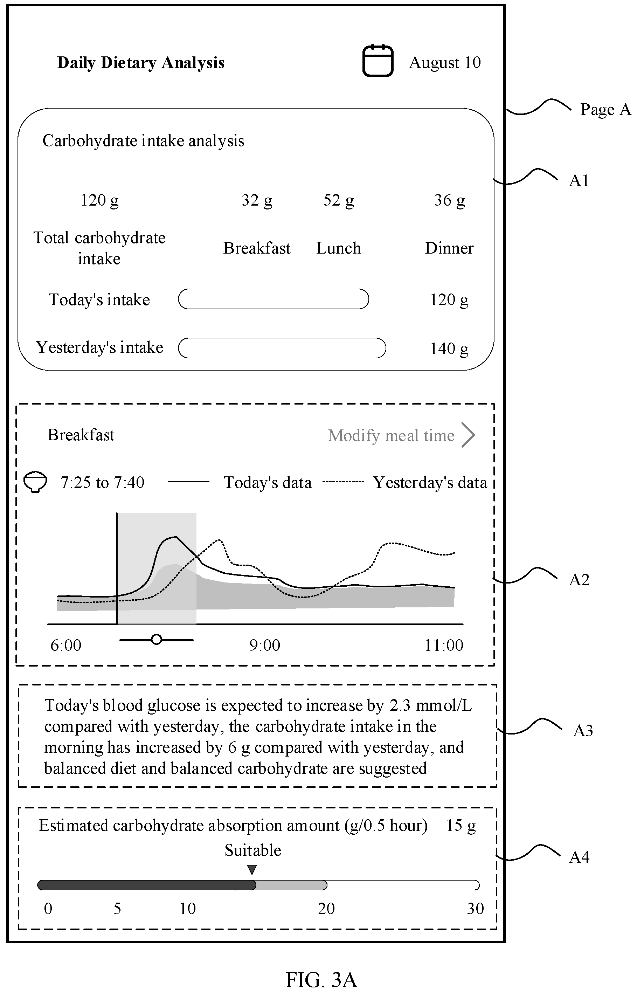

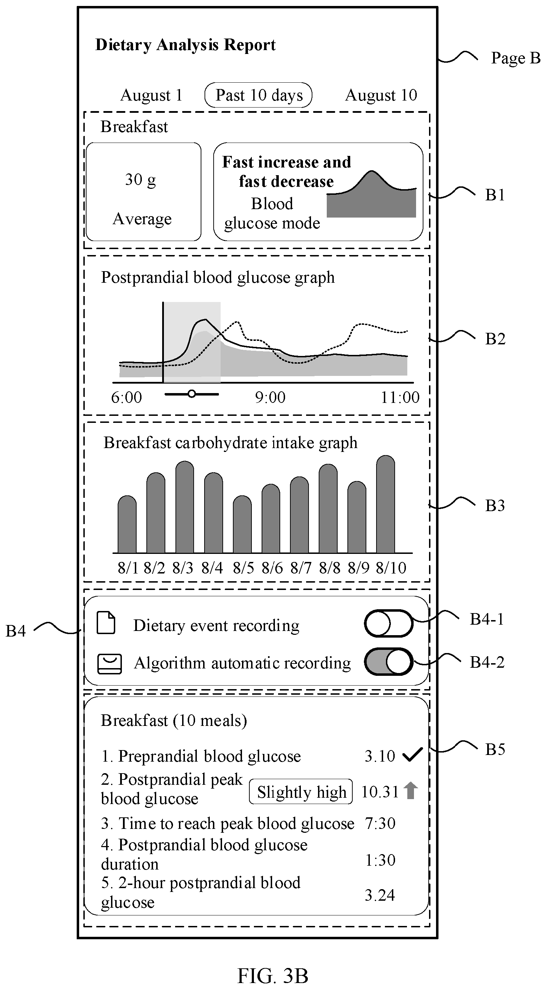

**For example, FIG. 3A and FIG. 3B show page content displayed by the electronic device 100 and used to evaluate the blood glucose health status of the user according to an embodiment of this application.**

FIG. 3A shows a page A according to an embodiment of this application. The page A may be used to display a daily dietary analysis result of the user by the electronic device 100.

As shown in FIG. 3A, the page A may include a block A1, a block A2, a block A3, and a block A4.

The block A1 may be used to display a carbohydrate intake of the user for a day, for example, may include carbohydrate intakes of the user at breakfast, lunch, and dinner for the day, and a total carbohydrate intake of the user for the day.

For example, the electronic device 100 may determine the carbohydrate intake of the user in the current dietary event by calculating an area under a blood glucose curve in a time period (for example, two hours) after the user starts the meal.

In addition, the block A1 may also display carbohydrate intakes of the user for two consecutive days. In this way, the user can learn about the change status of the carbohydrate intake of the user by comparing the carbohydrate intakes over the two consecutive days.

The block A2 may be used to display a postprandial blood glucose curve of the user, for example, display a blood glucose curve of the user after breakfast.

For example, after determining the meal time of the user, the electronic device 100 may collect a plurality of blood glucose values of the user within preset duration after the meal time, to obtain a postprandial blood glucose curve of the user, and annotate the blood glucose curve with the meal time of the user.

The block A3 may be used to display an opinion, a suggestion, and the like provided by the electronic device 100 for the user based on blood glucose and the carbohydrate intake of the user. For example, as shown in FIG. 3A, the block A3 may display "Today's blood glucose is expected to increase by 2.3 mmol/L compared with yesterday, the carbohydrate intake in the morning has increased by 6 g compared with yesterday, and balanced diet and balanced carbohydrate are suggested".

The block A4 may be used to display a carbohydrate absorption amount estimated by the electronic device 100 for the user. The carbohydrate absorption amount may indicate a food absorption condition of the user. If the user exhibits a greater increase in blood glucose after ingesting a food, food absorption of the user is better; or if the user exhibits a smaller increase in blood glucose after ingesting a food, food absorption of the user is poorer. For example, the electronic device 100 may determine the carbohydrate absorption amount based on an area under a blood glucose curve in a time period after the user has the meal. For example, as shown in FIG. 3A, the carbohydrate absorption amount estimated by the electronic device 100 for the user is 15 g/0.5 hour.

It can be learned from FIG. 3A that the electronic device 100 may summarize and analyze a dietary status and a blood glucose status of the user on a single day, and associate the diet of the user on the day with the blood glucose of the user. This helps the user find an association relationship between the diet and a blood glucose change, so that the user controls blood glucose based on the diet, thereby improving a capability of the user to control blood glucose of the user.

FIG. 3B shows a page B according to an embodiment of this application. The page B may be used to display results of dietary analysis performed by the electronic device 100 for the user over a plurality of days.

As shown in FIG. 3B, the page B may include a block B1, a block B2, a block B3, a block B4, and a block B5.

The block B1 may be used to display an average carbohydrate intake of the user in the same dietary event over a plurality of days. For example, FIG. 3B shows that an average carbohydrate intake of the user at breakfast over 10 days is 30 g.

The block B2 may be used to display a postprandial blood glucose graph of the same dietary event of the user over a plurality of days. The postprandial blood glucose graph may be used to display a blood glucose fluctuation of the user in a time period (for example, two hours) after the meal time.

The block B3 may be used to display a carbohydrate intake graph of the user. The carbohydrate intake graph may be used to display a carbohydrate intake of the user in the same dietary event (for example, a breakfast event) on each of the plurality of days. In this way, by using the carbohydrate intake graph, the user may learn about a change status of the carbohydrate intake of the dietary event of the user in a time period.

The block B4 may be used to display one or more function options provided by the electronic device 100. The electronic device 100 may detect a user operation performed by the user on a switch in the function option, and enable or disable a corresponding function. For example, as shown in FIG. 3B, the block B4 may include a switch B4-1. The switch B4-1 may be used to enable or disable a dietary event recording function. The dietary event recording function may be used to record a dietary event of the user. The block B4 may further include a switch B4-2. The switch B4-2 may be used to enable or disable an algorithm automatic recording function. The algorithm recording function may be used to trigger the electronic device 100 to automatically record the dietary event of the user by using an algorithm.

The block B5 may be used to display feature information obtained by the electronic device 100 by calculating related blood glucose values of the user in the same dietary event over a plurality of days, for example, preprandial blood glucose, postprandial peak blood glucose, time to reach peak blood glucose, postprandial blood glucose duration, and 2-hour postprandial blood glucose. The time to reach peak blood glucose may be a time obtained through calculation relative to the meal time predicted by the electronic device 100, or may be a time obtained through calculation relative to an objective and actual meal time. For example, the time to reach peak blood glucose may be presented as a time interval relative to the meal time, for example, half an hour, indicating that the blood glucose of the user reaches a peak in half an hour after the meal, or may be presented as a specific time point, for example, 8:00, indicating that the blood glucose of the user reaches a peak at 7:30. In this way, the electronic device 100 may quantify a preprandial blood glucose status and a postprandial blood glucose status of the user into a plurality of types of feature information, and evaluate the postprandial blood glucose health of the user, to help the user better learn about a physical condition of the user.

It can be learned from FIG. 3B that the electronic device 100 may summarize and analyze a dietary status and a blood glucose status of the user over a plurality of days, and associate the diet of the user on the plurality of days with the blood glucose of the user. This helps the user find an association relationship between the diet and a blood glucose change in a time period, so that the user can summarize a dietary pattern and control blood glucose based on the diet, thereby further improving the capability of the user to control blood glucose of the user.

It may be understood that FIG. 3A and FIG. 3B may be application pages provided by an application on the electronic device 100. The user may control the diet and blood glucose of the user by using the application. The user may intuitively learn the diet and blood glucose status of the user on a day by using the page A shown in FIG. 3A, and intuitively learn about the diet and blood glucose status of the user over a plurality of days by using the page B shown in FIG. 3B.

It should be noted that the pages shown in FIG. 3A and FIG. 3B are merely examples for description, and do not constitute a limitation on this embodiment of this application.

It can be learned from steps S101 to S104 that the electronic device 100 may search for a causal relationship between the dietary event of the user and a blood glucose concentration by using the meal time of the user as a starting point, so that a blood glucose change of the user and a diet adjustment can form a positive feedback. This helps the user improve the capability of the user to control blood glucose based on daily diet.

**FIG. 4 is a schematic flowchart for determining a meal time of a user according to an embodiment of this application.**

**S201:** An electronic device 100 determines a historical meal time t1 of a user based on historical blood glucose values of the user.

For example, the historical meal time t1 may be a time point or a time period.

The electronic device 100 may collect the blood glucose values of the user by using an electronic device 200. The historical blood glucose values may be blood glucose values of the user in a historical preset time period, for example, blood glucose values in past seven days. The historical meal time t1 may include a meal time that is of one or more dietary events and that is determined by the electronic device 100 based on the historical blood glucose values.

Usually, a blood glucose value of a user gradually increases and exceeds a specific value during a meal. In view of this phenomenon, it may be determined, based on whether a rate of increase in the blood glucose values of the user exceeds a threshold, whether the user has a dietary event. If the rate of increase in the blood glucose values of the user exceeds the threshold, preset duration before and after the blood glucose value is collected is a meal time period of the user.

For example, whether the user has a dietary event may be determined by using the following formula 1:

$$\frac{G_{t+\Delta t} - G_t}{\Delta t} > G \qquad \text{formula 1}$$

In the formula, $G_t$ represents a blood glucose value of the user at a historical time point t, $\frac{G_{t+\Delta t} - G_t}{\Delta t}$ represents a rate of increase in the blood glucose values of the user at the historical time point t, and G represents the threshold.

If the rate of increase in the blood glucose values of the user at the historical time point t exceeds the threshold, a time period [t-t0, t+t0] is the meal time period of the user. For example, t0 may be 15 minutes, 30 minutes, 1 hour, or the like. For example, if t0 is 15 minutes, and the rate of increase in the blood glucose values of the user at 11:30 exceeds the threshold, 11:15 to 11:45 may be the meal time period of the user.

In some implementations, if the historical meal time t1 is a time period, the electronic device 100 may determine the meal time period as the historical meal time t1.

In some other implementations, if the historical meal time t1 is a time point, the electronic device 100 may find the historical meal time t1 of the user in the meal time period.

For example, the electronic device 100 may directly determine a starting point of the meal time period as the meal time t1 of the user, or the electronic device 100 may further search for the meal time t1 with reference to a blood glucose value of the user in the time period. For example, in the time period, a time point at which the blood glucose value of the user first shows an increasing trend in chronological order is the historical meal time t1 of the user.

It may be understood that the electronic device 100 may alternatively determine the historical meal time t1 of the user in another manner. This is not limited in this embodiment of this application.

**S202:** The electronic device 100 determines a historical meal time t2 based on any one or more of the following: a historical meal time input by the user, a hand movement of the user, and a heart rate of the user.

For example, the historical meal time t2 may be a time point or a time period.

The electronic device 100 may provide an entry for the user to input the historical meal time, and the user manually inputs a historical meal time of a historical user event. The meal time may include one or more meal times. In other words, the electronic device 100 may obtain one or more historical meal times input by the user.

In some implementations, if a plurality of historical meal times are input by the user, after the electronic device 100 obtains the historical meal times input by the user, the electronic device 100 may further process the historical meal times, and determine, from the plurality of historical meal times, a historical meal time t2 representing a historical dietary event of the user.

For example, the electronic device 100 may normalize, by using preset duration (for example, half an hour) as a dimension, the plurality of historical meal times input by the user, extract a historical meal time with a highest occurrence frequency from the plurality of historical meal times, and use this historical meal time as the historical meal time t2 representing the historical dietary event of the user. For example, if the historical meal times input by the user are 11:10, 11:34, 11:45, 11:50, and 12:10, the plurality of historical meal times may be normalized into 11:00, 11:30, 11:30, 11:30, and 12:00. It may be learned that 11:30 is a meal time with a highest occurrence frequency. Therefore, 11:30 may be determined as the historical meal time t2 in the historical dietary event of the user.

For example, the electronic device 100 may obtain, by summing and averaging all historical meal times input by the user, the historical meal time t2 representing the historical dietary event of the user.

In addition, because a hand movement, for example, a movement of swinging up and down, is usually involved during a meal, if the electronic device 100 is a wearable device worn on a hand of the user, or the electronic device 100 is connected to a wearable device worn on a hand, the electronic device 100 may obtain a historical meal time determined by the wearable device based on the hand movement of the user. For example, if the wearable device detects that the hand of the user performs a repeated preset movement in a time period, the wearable device may determine, as a meal time, a time when the user first initiates the movement, and save the meal time in the electronic device 100. In this way, when the electronic device 100 needs to predict a future meal time of the user based on historical data, the electronic device 100 may perform prediction by using the historical meal time that is historically collected and that is determined based on the hand movement of the user.

The historical meal time that is determined based on the hand movement of the user and that is obtained by the electronic device 100 may also include one or more meal times. For example, the hand movement may include but is not limited to one or more of the following: a movement amplitude, a movement track, a movement frequency, and the like.

In some implementations, if a plurality of historical meal times are determined based on the hand movement of the user, the electronic device 100 may also obtain, based on the plurality of historical meal times, the historical meal time t2 representing the historical dietary event of the user.

Similarly to determining the historical meal time based on the hand movement of the user, because the heart rate usually changes when the user has a meal, if the electronic device 100 is a wearable device, or the electronic device 100 is connected to a wearable device, the electronic device 100 may obtain the historical meal time determined by the wearable device based on the heart rate. For example, if the wearable device detects that a heart rate change rate of the user is greater than a threshold, the wearable device may determine a current time as a meal time, and save the meal time in the electronic device 100. In this way, when the electronic device 100 needs to predict the future meal time of the user based on the historical data, the electronic device 100 may perform prediction by using the historical meal time that is historically collected and that is determined based on the heart rate of the user.

The historical meal time that is determined based on the heart rate of the user and that is obtained by the electronic device 100 may also include one or more meal times.

In some implementations, if a plurality of historical meal times are determined based on the heart rate of the user, the electronic device 100 may also obtain, based on the plurality of historical meal times, the historical meal time t2 representing the historical dietary event of the user.

It may be understood that if the historical meal time t2 obtained by the electronic device 100 includes any two or more of the meal time input by the user, the meal time determined based on the hand movement of the user, and the meal time determined based on the heart rate of the user, the electronic device 100 may determine, based on any plurality of the obtained meal times, the historical meal time t2 representing the historical dietary event of the user. For a specific manner in which the electronic device 100 determines, based on the plurality of historical meal times, the historical meal time t2 representing the historical dietary event of the user, refer to content about determining, based on the plurality of historical meal times input by the user, the historical meal time t2 representing the historical user event of the user. Details are not described herein again.

In some implementations, the historical meal times obtained in step S201 and step S202 may be meal times of historical dietary events that occur in a same historical time period. In this way, it can be ensured that the electronic device 100 can more accurately predict the future meal time by using an association between blood glucose and meals of the user in the same time period.

It may be understood that, in addition to determining the historical meal time t2 based on the meal time input by the user, the hand movement of the user, and the heart rate of the user, the electronic device 100 may further determine the historical meal time t2 by using other information. For example, the electronic device 100 may determine the historical meal time t2 by using physiological information of the user, including but not limited to one or more of the following: skin temperature, respiration, heart rate variability, blood pressure, blood oxygen, and the like. For another example, the electronic device 100 may determine the historical meal time t2 by using a location of the user. The electronic device 100 may determine, based on the location of the user, whether the user is within a range of a dining place such as a dining room or a restaurant, to determine the historical meal time t2. Alternatively, the electronic device 100 may determine, based on the location of the user, a room in which the user is located, to determine whether the user is in a dining room or another room in which a meal can be taken, to determine the historical meal time t2, and so on. A manner in which the electronic device 100 determines the historical meal time t2 is not limited in this application.

S203: The electronic device 100 determines a meal time t0 of the user based on the historical meal time t1 and the historical meal time t2.

The historical meal time t1 may include one or more meal times, and the historical meal time t2 may also include one or more meal times.

In some implementations, the electronic device 100 may determine, as the meal time t0 of the user, a time point that appears most frequently in the historical meal time t1 and the historical meal time t2.

In some other implementations, the electronic device 100 may determine the meal time t0 of the user by summing and averaging the historical meal time t1 and the historical meal time t2.

In some other implementations, the electronic device 100 may further determine the meal time t0 of the user by inputting the historical meal time t1 and the historical meal time t2 into a specified model. The specified model may be a model obtained through training by using a neural network algorithm and a large amount of known training data of the user at the meal time t0 after a time point and the historical meal time t1 and the historical meal time t2 before the time point.

It may be understood that the electronic device 100 may alternatively determine the meal time t0 of the user in another manner. This is not limited in this embodiment of this application.

S204: When the meal time t0 of the user is reached, the electronic device 100 outputs prompt information, where the prompt information is used to prompt the user that the predicted meal time has been reached currently.

After the electronic device 100 determines the meal time t0 of the user, the electronic device 100 may output prompt information (for example, first prompt information) when the meal time t0 of the user is reached, to prompt the user that the predicted meal time has been reached currently. For example, the electronic device 100 may output the prompt information by displaying the prompt information on a display, playing the prompt information via audio, or the like.

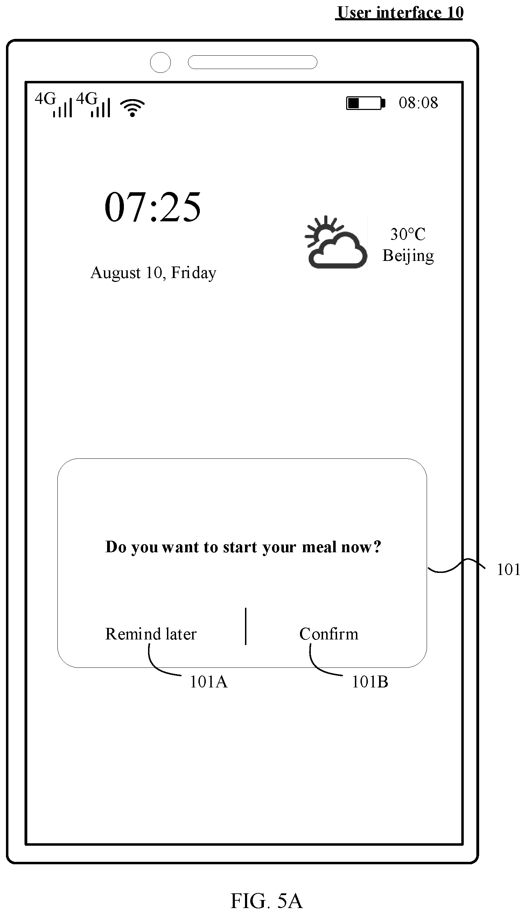

In some implementations, the electronic device 100 may detect an operation (for example, a first operation) performed on the prompt information, and determine the meal time t0 of the user as a meal time of one dietary event (for example, a first dietary event) of the user, or determine a meal time t3 of the user as a meal time of one dietary event of the user, where the meal time t3 of the user may be a time input by the user, or a time determined based on the meal time t0. For example, the meal time t3 of the user may be a time obtained by adding preset duration to the meal time t0. For details, refer to related descriptions in FIG. 5A.

This is because the meal time point t0 of the user that is predicted by the electronic device 100 may not be an actual meal time of the user. Therefore, the electronic device 100 may output prompt information when the meal time t0 of the user is reached, so that the user may correct the meal time of the user based on the prompt information, and that the electronic device 100 can provide a more accurate blood glucose evaluation result based on the actual meal time of the user.

If the electronic device 100 determines the meal time t3 of the user as a meal time of one dietary event of the user, after the electronic device 100 detects an operation performed on the prompt information, the electronic device 100 may display the prompt information again after a preset time interval. After detecting an operation (for example, a second operation) performed on the first prompt information, the electronic device 100 may determine the current time as the meal time t3 of the user.

For example, the prompt information may include two options: an option A and an option B. The option A may be used to trigger the user to determine the current time as an actual meal time of the user, and the option B is used to trigger the electronic device 100 to display the prompt information again after a time period (for example, 5 minutes). The first operation may be an operation performed on the option B, and the second operation may be an operation performed on the option A. For a detailed description of the prompt information, refer to FIG. 5A subsequently.

In other words, after the electronic device 100 outputs the prompt information to prompt the user that the predicted meal time has been reached currently, if the current time is not the actual meal time of the user, the user may control the electronic device 100 to delay displaying the prompt information for a period of time. When the actual meal time is reached, the user may confirm the prompt information, so that the electronic device 100 determines the current time as the actual meal time of the user, thereby recording the actual meal time of the user.

It may be understood that if the actual meal time of the user is still not reached, the user may allow the electronic device 100 to delay displaying the prompt information for multiple times, so that the electronic device 100 may repeatedly display the prompt information multiple times until the user determines the meal time.

Further, before or after the electronic device 100 determines a meal time of one dietary event of the user, the electronic device 100 may determine a food (for example, a first food) to be ingested by the user and an intake (for example, a first intake) of the food in the dietary event, determine a blood glucose curve (for example, a first blood glucose curve) based on information about the food, the intake of the food, and a blood glucose value of the user before the meal time t0 of the user, for example, a current time point, and display the blood glucose curve to the user for viewing, where the blood glucose curve is a blood glucose change of the user, predicted by the electronic device 100, under influence of the dietary event.

In this way, before the user has a meal, the user may view a blood glucose change that is predicted by the electronic device 100 and that may occur after the user has the meal, so that the user can adjust, in a timely manner, the food to be ingested and the intake of the food in this dietary event, and that the user can take a timely measure before the dietary intake is to endanger physical health of the user, to ensure the physical health of the user.

Further optionally, the electronic device 100 may further determine a blood glucose curve (for example, a second blood glucose curve) based on a blood glucose value of the user before the current time point, and display the blood glucose curve to the user for viewing. The blood glucose curve is a blood glucose change of the user, predicted by the electronic device 100, in absence of the dietary event.

In this way, the user may compare a future blood glucose change of the user under the influence of the dietary event with a future blood glucose change of the user in absence of the dietary event, to highlight an association between the dietary event and the blood glucose of the user, so that the user better plans a healthy dietary habit, thereby effectively avoiding risks caused by hyperglycemia and hypoglycemia.

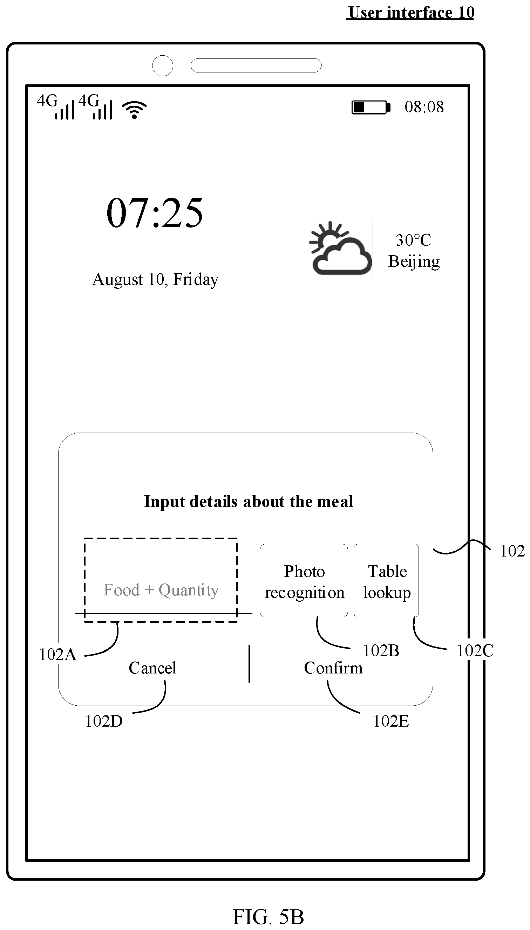

For details about a related user interface for predicting future blood glucose by the electronic device 100, refer to related content in FIG. 5B to FIG. 5D subsequently.

In a process in which the electronic device 100 determines the blood glucose curve by using the information about the food, the intake of the food, and the blood glucose value of the user before the meal time t0 of the user, the electronic device 100 may determine the blood glucose curve by using an absorption index of the food for the user, the intake of the food, and the blood glucose value of the user before the current time point. The absorption index of the food is related to the food and a physical condition of the user.

The physical condition may include any one or more of the following: an age, a height, a weight, a disease type, and medication information.

For example, the electronic device 100 may predict a postprandial blood glucose value of the user by inputting the absorption index of the food for the user, the intake of the food, and a preprandial blood glucose value, for example, before the current time point, into the specified model, and then connect the predicted blood glucose values in chronological order to generate a curve, to obtain a postprandial blood glucose curve of the user. The specified model may be a model obtained through training by using data such as known preprandial and postprandial blood glucose values, the absorption index of the food for the user, and the intake of the food.

In addition, the absorption index of the food for the user is an indicator used to measure a rate and a capability of blood glucose increase after the user consumes the food. A food with a high absorption index is digested quickly in the gastrointestinal tract and absorbed at a high rate, leading to a fast release of glucose and a high peak in blood glucose after the glucose enters the bloodstream, that is, a great blood glucose increase. A food with a low absorption index stays in the gastrointestinal tract for a long time and is absorbed at a low rate, leading to a slow release of glucose, a low peak after the glucose enters the bloodstream, and a slower decline in blood glucose. The user may appropriately arrange meals and adjust a blood glucose concentration of the user based on absorption indexes of different foods.

Because different users have different absorption conditions for different foods, the electronic device 100 may customize, for the user, absorption indexes of different foods for the user, and store the customized indexes locally. Therefore, the electronic device 100 needs to predict a postprandial blood glucose value of the user before the user has the meal. The electronic device 100 may display different foods and absorption indexes of different foods for the user to the user for viewing, and after the user selects a specified food, predict a future blood glucose status of the user based on an absorption index of the food for the user, and display the future blood glucose status to the user for viewing. The electronic device 100 may determine the absorption index of the food for the user based on a blood glucose value of the user in a time period (for example, first duration) and the physical condition of the user after the user ingests a specified intake (for example, a second intake) of the food.

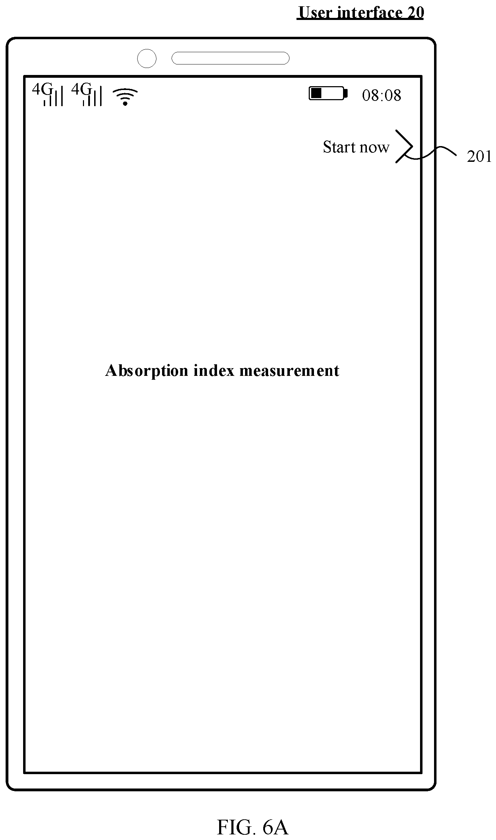

For a specific process in which the electronic device 100 measures absorption indexes of different foods for the user, refer to related descriptions in FIG. 6A to FIG. 6F subsequently. Details are not described herein.

In some other implementations, after the electronic device 100 obtains the meal time t0 of the user through calculation, the electronic device 100 may determine, based on a blood glucose value of the user in a time period (for example, second duration) after the meal time t0 of the user, an intake (for example, a third intake) of the user in this dietary event (for example, the first dietary event), and display the intake to the user for viewing. In this way, the user can learn about the intake of each meal, and control the intake of each meal, so that the user can develop a regular dietary habit and control a stable fluctuation of blood glucose.

In some other implementations, after the electronic device 100 obtains the meal time t0 of the user through calculation, the electronic device 100 may obtain blood glucose values of the user within preset duration (for example, third duration) after the meal time t0 of the user, generate a blood glucose curve (for example, a third blood glucose curve) by using the blood glucose values, and display the blood glucose curve to the user for viewing. The blood glucose curve may be determined based on a plurality of blood glucose values of the user within the preset duration after the meal time point t0.

In this way, after the user has the meal, the user can learn about an actual blood glucose status of the user under influence of the dietary event. This helps the user learn about influence of diet on blood glucose more intuitively, so that the user can control blood glucose based on the diet.

**To help better understand an interaction process between the electronic device 100 and the user in this solution, some user interfaces that may exist on the electronic device 100 are hereinafter described with reference to FIG. 5A to FIG. 5D and FIG. 6A to FIG. 6F.**

**For example, FIG. 5A to FIG. 5D show some user interfaces displayed by the electronic device 100 after the meal time t0** of the **user is reached according to an embodiment of this application.**

As shown in FIG. 5A, a user interface 10 may include prompt information 101. The prompt information 101 may be prompt information displayed by the electronic device 100 when the meal time t0 of the user is reached. The prompt information 101 may be used to prompt the user that the predicted meal time has been reached currently.

The prompt information 101 may include a remind later option 101A and a confirm option 101B. If the electronic device 100 detects a user operation performed by the user on the remind later option 101A, for example, a tap operation, it indicates that the current time is not an actual meal time of a dietary event of the user. Therefore, the electronic device 100 may display the prompt information 101 later. If the electronic device 100 detects a user operation performed by the user on the confirm option 101B, for example, a tap operation, it indicates that the current time is an actual meal time of a dietary event of the user in deed. Therefore, the electronic device 100 may determine the current time point as an actual meal time of the user.

For example, if the electronic device 100 detects a user operation performed on the remind later option 101A, the electronic device 100 may display the prompt information 101 at an interval of preset duration (for example, 5 minutes). If the electronic device 100 detects a user operation performed by the user on the confirm option 101B when the electronic device 100 displays the prompt information 101 again 5 minutes later, the electronic device 100 may determine a time point, 5 minutes later than the meal time t0 of the user and obtained by the electronic device 100 through calculation, as an actual meal time of the user, thereby correcting the meal time t0 of the user.

In some implementations, the prompt information 101 may further include a time modification option. The user may manually set the actual meal time of the user by using the time modification option. The actual meal time may be earlier than the current time, or may be later than the current time. In this way, the electronic device 100 can be prevented from repeatedly displaying the prompt information 101, so that the user can correct the meal time of the user in a single attempt. In addition, if the meal time t0 of the user that is predicted by the electronic device 100 is later than the actual meal time of the user, the user may also determine, as the actual meal time of the user in a manual input and setting manner, a time earlier than the meal time t0 of the user.

In some other implementations, the prompt information 101 may further include a delay option. The delay option may be used to trigger the electronic device 100 to automatically use a time that is later than the meal time t0 of the user by preset duration (for example, 5 minutes) as the actual meal time of the user.

If the electronic device 100 detects a user operation performed by the user on the confirm option 101B, for example, a tap operation, the electronic device 100 may update, in response to the operation, the prompt information 101 shown in FIG. 5A to prompt information 102 shown in FIG. 5B. The prompt information 102 may be used to prompt the user to input details about the meal.

It may be understood that, after the meal time point t0 is reached, the electronic device 100 may first display the user interface 10 shown in FIG. 5B, to prompt the user to input details about the meal, and then display the user interface 10 shown in FIG. 5A, to prompt the user to confirm an actual meal time in this dietary event. Alternatively, the prompt information 101 shown in FIG. 5A may further include a prediction option. When the meal time t0 of the user is reached, the electronic device 100 may display the prompt information 101. If the electronic device 100 detects a user operation performed on the prediction option, the electronic device 100 may display the prompt information 101 shown in FIG. 5B. In this way, when the meal time t0 of the user that is predicted by the electronic device 100 is reached, the user may not first confirm the meal time of the user, but first predict a future blood glucose change of the user by inputting details about the current meal of the user, and then determine the meal time of the user.

As shown in FIG. 5B, the prompt information 102 may include a text input box 102A, a photo recognition option 102B, a table lookup option 102C, a cancel option 102D, and a confirm option 102E.

The text input box 102A may be used to input a food to be ingested by the user and a quantity thereof in this dietary event. The electronic device 100 may detect a user operation performed by the user on the text input box 102A, for example, a tap operation, and additionally display a keyboard on the user interface 10. The user may manually input, in the text input box 102A by using the keyboard, the food to be ingested and the quantity thereof in this dietary event.

The photo recognition option 102B may be used to trigger the electronic device 100 to automatically recognize, in a manner of photographing the food for this meal, the food to be ingested and the quantity thereof in this dietary event, and input recognized information into the text input box 102A for display to the user.

The table lookup option 102C may be used to trigger the electronic device 100 to display a plurality of foods that can be selected by the user and absorption indexes thereof. The user may select, from the plurality of foods, the food to be ingested by the user in this dietary event.

In this embodiment of this application, considering that different users have different food absorption effects, the electronic device 100 may define a customized food absorption index table for the user. Absorption indexes of the foods may be obtained by the electronic device 100 through calculation based on blood glucose changes of the user after the user eats the foods to be measured. For a specific manner of calculating a food absorption index, and a user interface displayed when the electronic device 100 measures the food absorption index, refer to subsequent content. Details are not described herein.

The cancel option 102D may be used to cancel an input of meal details. For example, when the electronic device 100 detects a user operation performed on the cancel option 102D, for example, a tap operation, the electronic device 100 may close the prompt information 102, or update the prompt information 102 to the prompt information 101 shown in FIG. 5A.

The confirm option 102E may be used to confirm the food and the quantity thereof that are input by the user, and trigger the electronic device 100 to predict a postprandial blood glucose change of the user based on the currently input food and quantity thereof.

It can be learned from FIG. 5B that, the user may manually input the food to be ingested and the quantity thereof in this dietary event, or may recognize, through photo recognition, the food to be ingested by the user and the quantity thereof, or may select, from a plurality of foods through table lookup, the food to be ingested by the user and the quantity thereof.

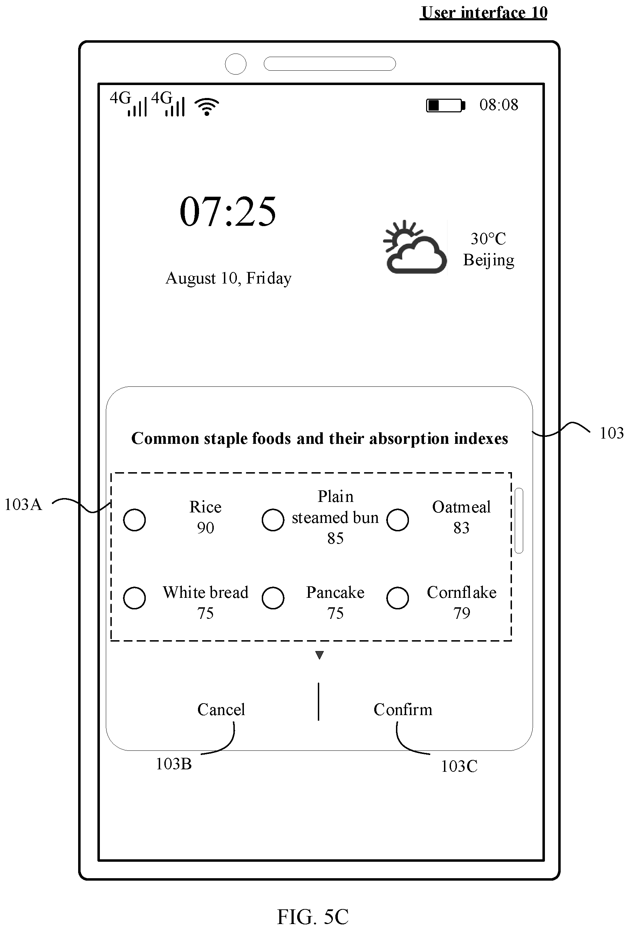

For example, when the electronic device 100 detects a user operation performed on the table lookup option 102C, for example, a tap operation, the electronic device 100 may display, in response to the operation, prompt information 103 shown in FIG. 5C. The prompt information 103 may be used to display the food absorption index table customized by the electronic device 100 for the user.

As shown in FIG. 5C, the prompt information 103 may include a food display section 103A, a cancel option 103B, and a confirm option 103C.

The food display section 103A may be used to display names and absorption indexes of one or more foods. The electronic device 100 may detect a selection operation performed on one of the foods, and select the food as the food to be ingested by the user in this dietary event.

The cancel option 103B may be used to cancel the selection, made through table lookup, the food that is to be ingested by the user. For example, when the electronic device 100 detects a user operation performed on the cancel option 103B, for example, a tap operation, the electronic device 100 may close the prompt information 103, or further switch from the prompt information 103 to the prompt information 102 shown in FIG. 5B.

The confirm option 103C may be used to confirm that the food selected by the user in the food display section 103A is used as a food that is to be ingested by the user in this dietary event.

It may be understood that, after the user selects the food based on the prompt information 103, the user may further set, based on the prompt information 103, a food quantity for the food selected by the user. A function that can be implemented by the prompt information 103 is not limited in this embodiment of this application.

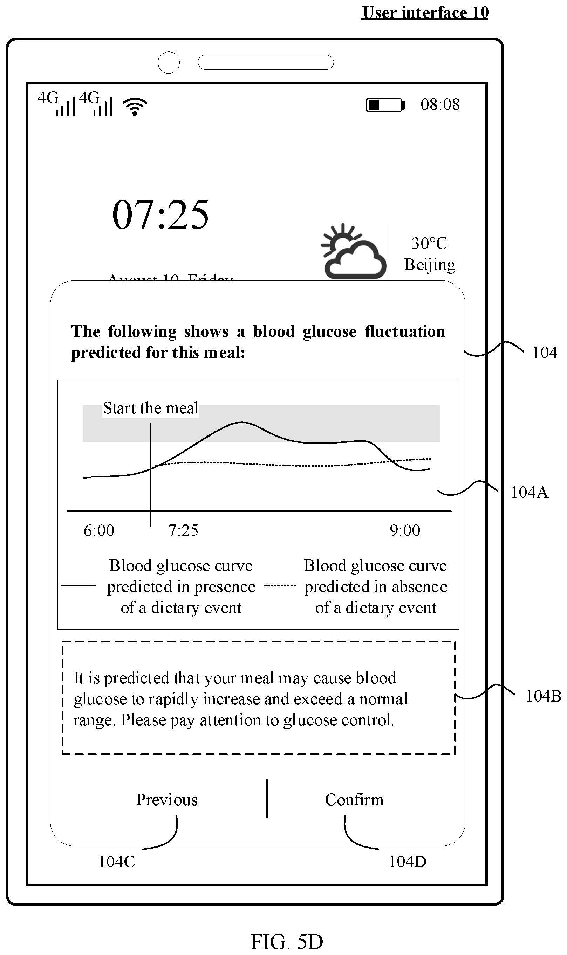

For example, after the electronic device 100 detects a user operation performed on the confirm option 103C, for example, a tap operation, the electronic device 100 may predict, based on the food and the quantity thereof that are input by the user and by using an absorption index of the food for the user, a future blood glucose curve of the user under influence of this dietary event, to display prompt information 104 shown in FIG. 5D. The prompt information 104 may be used to display a future blood glucose status of the user that is predicted by the electronic device 100.

As shown in FIG. 5D, the prompt information 104 may include a blood glucose display area 104A, an opinion display area 104B, a previous option 104C, and a confirm option 104D.

The blood glucose display area 104A may be used to display a future blood glucose curve of the user, predicted by the electronic device 100 based on the absorption index of the food selected by the user, under influence of this dietary event. Further optionally, the blood glucose display area 104A may be further used to display a future blood glucose curve of the user, predicted by the electronic device 100, in absence of this dietary event. In this way, the user may learn about future blood glucose changes of the user in presence of this dietary event and in absence of this dietary event by using the blood glucose display area 104A, to highlight an association between the dietary event and the blood glucose of the user, so that the user better plans a healthy dietary habit, thereby effectively avoiding risks caused by hyperglycemia and hypoglycemia.

The opinion display area 104B may be used to display some opinions and suggestions provided by the electronic device 100 based on a future blood glucose status of the user. For example, the opinion display area 104B may display "It is predicted that your meal may cause blood glucose to rapidly increase and exceed a normal range. Please pay attention to glucose control." It may be understood that the electronic device 100 may further display another opinion or suggestion in the opinion display area 104B, for example, suggesting a food to be used by the user for the meal or not suggesting a food to be used by the user for the meal.

The previous option 104C may be used to trigger the electronic device 100 to switch from the currently displayed prompt information 104 back to the prompt information 103 shown in FIG. 5C or the prompt information 102 shown in FIG. 5B.

The confirm option 104D may be used to trigger the electronic device 100 to close the prompt information 104.

It can be learned from FIG. 5A to FIG. 5D that, when the predicted meal time t0 of the user is reached, the electronic device 100 may display prompt information, to prompt the user whether to start the meal currently. If the user chooses to start the meal, the user may be prompted to input a meal intake of the user, so that the electronic device 100 can predict a future blood glucose value of the user based on the meal time, and that the user can control a blood glucose fluctuation of the user based on the diet.

In this embodiment of this application, the electronic device 100 may further measure an absorption index of a food ingested by the user, and customize a food absorption index library for the user, to help the user measure a glucose increase resulting from different foods at home, so that the user controls intakes of various foods by referring to the food absorption index library, thereby achieving a good glucose control effect.

For example, measuring an absorption index of a food usually includes the following steps:

(1) Keep fasting for a period of time before measurement.

For example, keep fasting for 8 to 10 hours before measurement. For example, the user may perform measurement at breakfast.

(2) During measurement, ingest a specific intake of the measured food and finish eating in a specified time period.

For example, add 75 g anhydrous glucose powder to 300 ml water and finish eating within 5 minutes.

(3) After finishing eating the food, calculate an absorption index of the measured food based on a blood glucose value in a time period (for example, 2 hours) after the user eats the food.

For example, the absorption index of the food may be calculated by using the following formula 2:

$$\text{Absorption index} = F(T, S, BG[], P[]) \qquad \text{formula (2)}$$

In the formula, T represents a food type, for example, rice, plain steamed bun, or oatmeal, S represents an intake of the food, BG[] represents a set of blood glucose values of the user in a time period after the user eats the food, P[] represents a user information set, where the user information is used to reflect the physical condition of the user, such as the age, height, weight, disease type, and medication information, and $F(\cdot)$ represents a meal model, where an input of the meal model includes T, S, BG[], and P[], and an output includes the absorption index. For example, the meal model may be obtained by training a large amount of known data related to foods, blood glucose, and the like by using a multiple linear regression algorithm. It may be understood that the meal model may alternatively be a neural network model. The meal model is not limited in this embodiment of this application.

It can be learned from the formula 2 that, in the absorption index measurement method provided in this embodiment of this application, different physical conditions of different users are considered, and user information is introduced in the absorption index measurement. If the user is a patient with diabetes and is taking medicine, in the method, an absorption index of a food for the user can be calculated by taking a medication effect and a pancreatic function of the patient into account, so that the food absorption index obtained through calculation is more consistent with an actual blood glucose absorption condition of the user, and that the user can design a better dietary plan.

In addition, it should be noted that, before measurement, during measurement, and after measurement, the electronic device 100 may further output related precautions for measuring the absorption index. For example, the electronic device 100 may prompt the user to maintain normal diet for a few days before measurement, with a daily carbohydrate intake not less than 150 g. For another example, the electronic device 100 may further prompt the user not to drink tea and coffee, not to smoke, not to perform strenuous exercise, and the like in the measurement process.

**FIG. 6A to FIG. 6F show some user interfaces used in a process of measuring an absorption index of a food by the electronic device 100 according to an embodiment of this application.**

FIG. 6A shows a user interface 20 displayed by the electronic device 100 after the electronic device 100 detects that the user starts to measure an absorption index of a food.

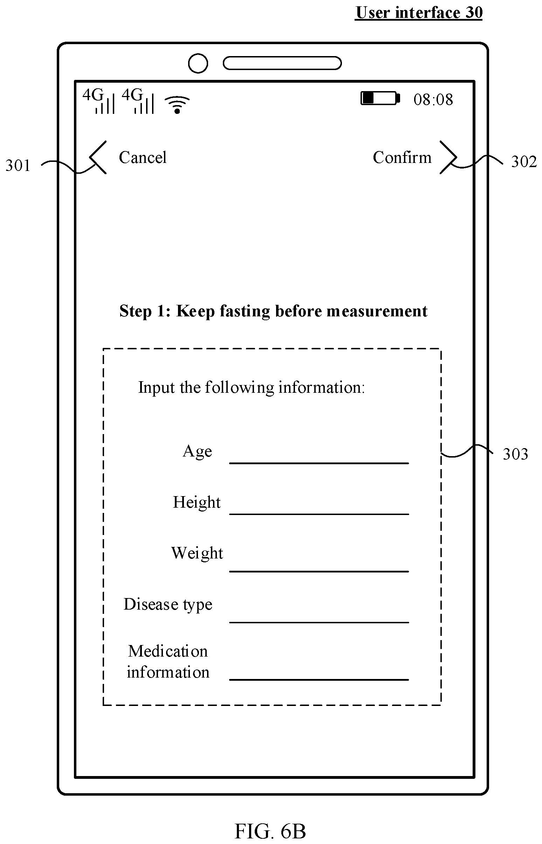

As shown in FIG. 6A, the user interface 20 may include a start measurement option 201. When the electronic device 100 detects a user operation performed on the start measurement option 201, for example, a tap operation, the electronic device 100 may display a user interface 30 shown in FIG. 6B. The user interface 30 may be used to display related content in step 1 of measuring the absorption index.

As shown in FIG. 6B, the user interface 30 may include a cancel option 301, a confirm option 302, and an information filling field 303.

The cancel option 301 may be used to trigger returning to a previous page, for example, returning to the user interface 20 shown in FIG. 6A.

The confirm option 302 may be used to proceed to a next step of measuring the absorption index after the user completes information filling in the information filling field 303.

The information filling field 303 may be used to display user information that needs to be filled in by the user. For example, the user information may include an age, a height, a weight, a disease type, medication information, and the like.

It may be understood that, if the user interface 30 is a user interface displayed by the electronic device 100 when the user measures the absorption index for the first time, the user interface 30 may include the information filling field 303 used to input user information. If the user interface 30 is a user interface displayed by the electronic device 100 when the user does not measure the absorption index for the first time, the user interface 30 may not include the information filling field 303, and the electronic device 100 directly uses the user information input when the user measures the absorption index for the first time, to calculate the food absorption index measured this time.

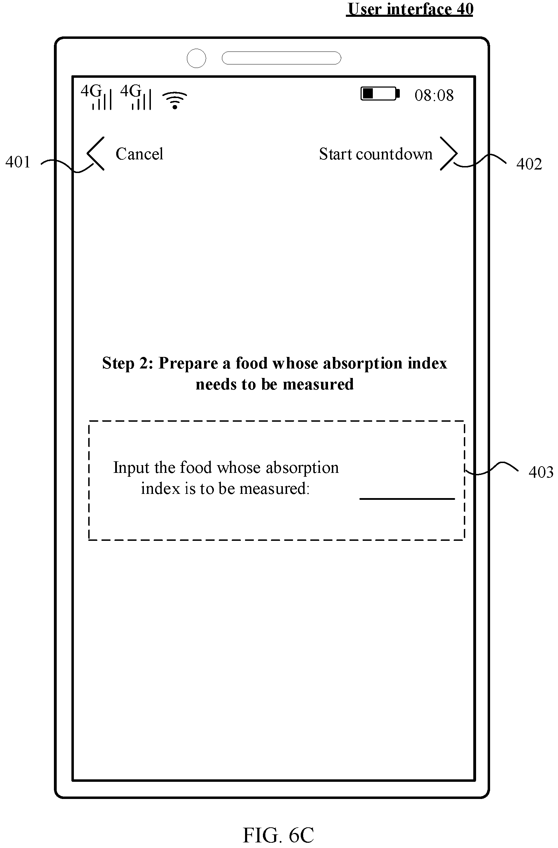

For example, after the user fills in the information filling field 303, if the electronic device 100 detects a user operation performed on the confirm option 302, for example, a tap operation, the electronic device 100 may display, in response to the operation, a user interface 40 shown in FIG. 6C. The user interface 40 may be used to display related content in step 2 of measuring the absorption index.

As shown in FIG. 6C, the user interface 40 may include a cancel option 401, a start countdown option 402, and a food filling field 403.

The cancel option 401 may be used to trigger returning to a previous page, for example, returning to the user interface 30 shown in FIG. 6B.

The start countdown option 402 is used to start a countdown.

The food filling field 403 may be used to display food information that needs to be filled in by the user for measuring the absorption index this time, for example, a name of the food.

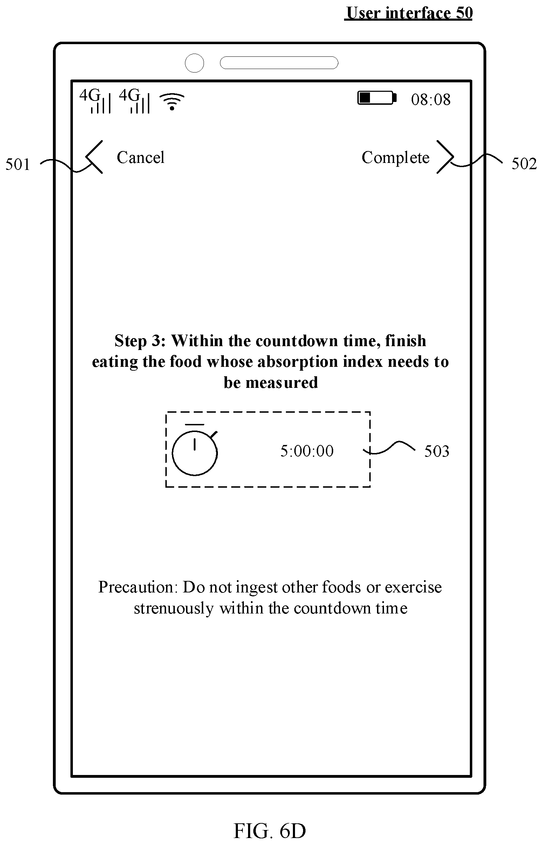

For example, after the user fills in the food name in the food filling field 403, if the electronic device 100 detects a user operation performed on the start countdown option 402, for example, a tap operation, the electronic device 100 may, in response to the operation, proceed to step 3 of measuring the absorption index, start a countdown, and display a user interface 50 shown in FIG. 6D. The user interface 50 may be used to display a countdown time, display prompt information, and prompt the user to finish eating, within the countdown time, the food whose absorption index needs to be measured.

As shown in FIG. 6D, the user interface 50 may include a cancel option 501, a complete option 502, and a countdown 503.

The cancel option 501 may be used to trigger returning to a previous page, for example, returning to the user interface 40 shown in FIG. 6C.

The complete option 502 may be used to trigger switching to a next page.

The countdown 503 may be used to display a countdown process of specified duration (for example, 5 minutes) after the display of the user interface 50 starts.

Optionally, the user interface 50 may further display precautions in the process of measuring the absorption index by the user. For example, as shown in FIG. 6D, the user interface 50 may include prompt information "Precaution: Do not ingest other foods or exercise strenuously within the countdown time."

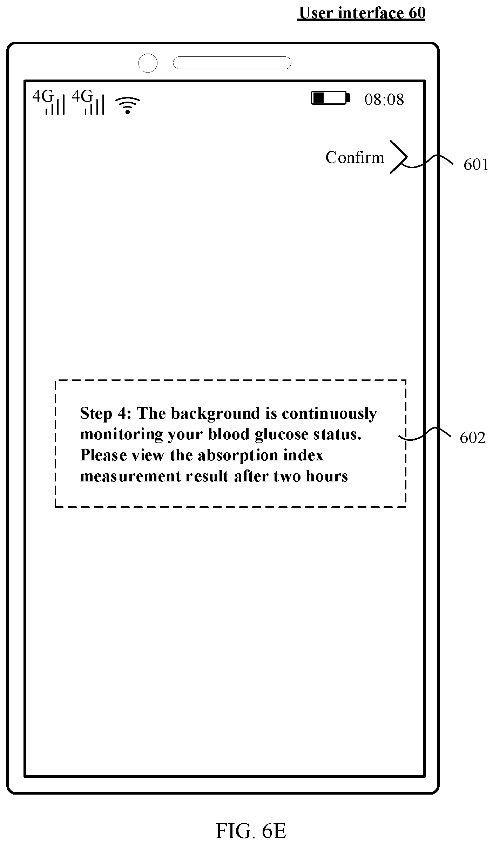

For example, if the user finishes eating, within the countdown time, the food whose absorption index needs to be measured, the electronic device 100 may detect a user operation performed on the complete option 502, for example, a tap operation, and the electronic device 100 may display, in response to the operation, a user interface 60 shown in FIG. 6E. The user interface 60 may be used to display related content in step 4 of measuring the absorption index.

As shown in FIG. 6E, the user interface 60 may include confirm information 601 and prompt information 602.

The confirm information 601 may be used to trigger closing of the user interface 60.

The prompt information 602 may be used to prompt the user to view an absorption index measurement result after a time period. For example, the prompt information 602 may include: "The background is continuously monitoring your blood glucose status. Please view the absorption index measurement result after two hours."

Then the electronic device 100 may obtain, through calculation based on the food measured by the user, the intake of the food, the user information, the blood glucose value of the user, and the foregoing formula 2, the food absorption index measured by the user.

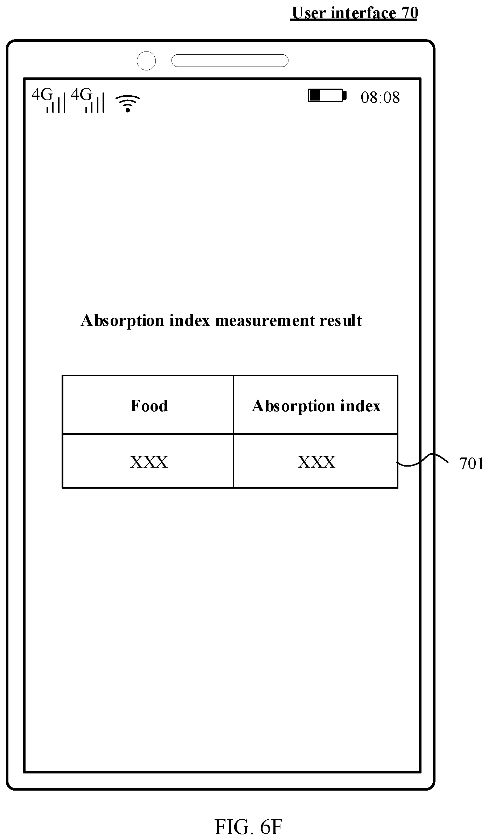

For example, FIG. 6F shows a user interface 70 displayed by the electronic device 100 after the electronic device 100 obtains, through calculation, the food absorption index measured by the user.

As shown in FIG. 6F, the user interface 70 may include a table 701. The table 701 may be used to display the name of the food measured by the user and the absorption index of the food.

Further, after the electronic device 100 obtains the absorption index of the food through measurement, the electronic device 100 may save the absorption index of the food locally. In addition, the electronic device 100 may organize absorption indexes of a plurality of foods measured by the user into a food absorption index library. By viewing the food absorption index library, the user may learn about a degree of influence of each food on blood glucose of the user, and predict blood glucose after eating a related food, to appropriately arrange diet.

It can be learned from FIG. 6A to FIG. 6F that the electronic device 100 may display prompt information related to absorption index measurement, to guide the user to complete food absorption index measurement at home, so as to help the user customize a food absorption index library that meets the physical condition of the user, so that the user formulates a better dietary plan.

It may be understood that FIG. 5A to FIG. 5D and FIG. 6A to FIG. 6F are merely examples for description, and do not constitute a limitation on this application.

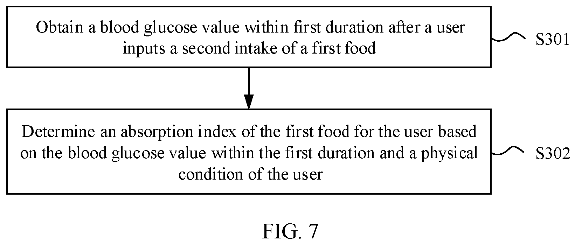

**FIG. 7 is a schematic flowchart of another blood glucose management method according to an embodiment of this application.**

As shown in FIG. 7, the method may include the following steps.

**S301:** An electronic device 100 obtains a blood glucose value within first duration after a user inputs a second intake of a first food.

The electronic device 100 may obtain the blood glucose value of the user by using an electronic device 200.

The second intake may be an intake that is preset when an absorption index of the food for the user is measured, for example, 50 g. The first duration may be duration preset when the absorption index of the food for the user is measured, for example, 2 hours. The first food may be any food, for example, rice, plain steamed bun, or oatmeal.

For example, the electronic device 100 may display a related user interface, to guide the user to measure the food absorption index at home, and in a measurement process, prompt the user with precautions related to measuring the absorption index.

For a specific user interface displayed by the electronic device 100 in a process of measuring the food absorption index, refer to FIG. 6A to FIG. 6F.

**S302:** The electronic device 100 determines the absorption index of the first food for the user based on the blood glucose value within the first duration and a physical condition of the user.

The physical condition of the user may include any one or more of the following: an age, a height, a weight, a disease type, medication information, and the like.

Specifically, the electronic device 100 may obtain the absorption index of the first food for the user by inputting a food type of the first food, the second intake, the blood glucose value within the first duration, and user information that reflects the physical condition of the user into a user model. The user model may be a model obtained by training a large amount of known data related to foods, blood glucose, and the like.

For detailed content about determining, by the electronic device 100, the absorption index of the first food for the user, refer to related content in the foregoing formula 2. Details are not described herein again.

In some implementations, after the electronic device 100 determines the absorption index of the first food for the user, if the electronic device 100 determines that a food to be ingested in a first dietary event is the first food, and determines that an intake of the first food is a first intake, the electronic device 100 may determine a first blood glucose curve based on the absorption index of the first food for the user, the first intake of the first food, and a blood glucose value of the user before a current time point, where the first blood glucose curve is a blood glucose change of the user, predicted by the electronic device 100, under influence of the first dietary event. The electronic device 100 may display the first blood glucose curve.

For example, the first blood glucose curve may be a blood glucose curve that is predicted under influence of a dietary event and that is displayed in the blood glucose display area 104A shown in FIG. 5D.

In this way, before the user has a meal, the electronic device 100 may predict, based on an absorption index and an intake of a food that is to be ingested by the user for this meal, and a historical blood glucose status of the user, a blood glucose status of the user under influence of the dietary event, so that the user controls a dietary intake in a timely manner before the dietary event influences a blood glucose fluctuation of the user, to ensure physical health of the user.

Further optionally, the electronic device 100 may further determine a second blood glucose curve based on a blood glucose value of the user before the current time point, where the second blood glucose curve is a blood glucose change of the user, predicted by the electronic device 100, in absence of the first dietary event. The electronic device 100 may display the second blood glucose curve.

For example, the second blood glucose curve may be a blood glucose curve that is predicted in absence of a dietary event and that is displayed in the blood glucose display area 104A shown in FIG. 5D.

In this way, the user may compare a future blood glucose change of the user under the influence of the dietary event with a future blood glucose change of the user in absence of the dietary event, to highlight an association between the dietary event and the blood glucose of the user, so that the user better plans a healthy dietary habit, thereby effectively avoiding risks caused by hyperglycemia and hypoglycemia.

In some implementations, the electronic device 100 may determine a first meal time based on historical blood glucose values of the user; determine a second meal time based on any one or more of the following: a meal time input by the user, a hand movement of the user, and a heart rate of the user; and determine a third meal time based on the first meal time and the second meal time, where that the electronic device 100 determines the first food to be ingested by the user and the first intake of the first food in the first dietary event may be that the electronic device 100 determines the first food to be ingested by the user and the first intake of the first food in the first dietary event after the third meal time is reached.

In other words, the electronic device 100 may summarize a meal pattern of the user in daily diet by using historical meal times estimated by the user in a plurality of manners, so as to accurately predict a meal time of the user, and after the meal time is reached, predict, based on a food to be ingested by the user, an intake, and a blood glucose status of the user, a blood glucose change that will occur in a current upcoming dietary event of the user, so that the user can control the dietary intake in a timely manner before the meal, and control a blood glucose fluctuation within a normal range.

The electronic device 100 may determine the first meal time based on a rate of increase in the historical blood glucose values of the user. For example, when the rate of increase in the blood glucose values is greater than a threshold, the electronic device 100 may trace back from a time point at which the blood glucose value is recorded, and determine a time point, at a specific interval from that time point, as a historical meal time of the user, that is, the first meal time.

It may be understood that the electronic device 100 may further determine the first meal time in another manner. For detailed content about determining, by the electronic device 100, the first meal time and the second meal time, and determining the third meal time based on the first meal time and the second meal time, refer to the foregoing descriptions about determining the historical meal time t1 and the historical meal time t2, and determining the meal time t0 of the user based on the historical meal time t1 and the historical meal time t2. Details are not described herein again.

Further, the electronic device 100 may output first prompt information when the third meal time is reached, where the first prompt information indicates that a current time reaches the meal time predicted by the electronic device 100.

In this way, based on the first prompt information, the user may learn that the meal time predicted by the electronic device 100 has been reached currently.

Further, after the electronic device 100 outputs the first prompt information, the electronic device 100 may detect a first operation performed on the first prompt information, and determine the third meal time as a meal time of the first dietary event, or determine a fourth meal time as a meal time of the first dietary event, where the fourth meal time is different from the third meal time.

The fourth meal time may be a time input by the user, or a time determined based on the third meal time. For example, the fourth meal time may be a time obtained by adding preset duration to the third meal time.

This is because the meal time predicted by the electronic device 100 may not be an actual meal time of the user. Therefore, after the predicted meal time is reached, the electronic device 100 may remind the user to confirm or modify the time, so that the electronic device 100 can provide a more accurate blood glucose evaluation result based on the actual meal time of the user.

In some implementations, if the electronic device 100 determines the fourth meal time as the meal time of the first dietary event of the user, after the electronic device 100 detects the first operation performed on the first prompt information, the electronic device 100 may display the first prompt information after a first time interval, and after detecting a second operation performed on the first prompt information, determine the current time as the fourth meal time.

For example, in this case, the first prompt information may be the prompt information 101 shown in FIG. 5A, the first operation may be an operation performed on the remind later option 101A, and the second operation may be an operation performed on the confirm option 101B.

In some implementations, after the electronic device 100 determines the third meal time, the electronic device 100 may further determine a third intake of the user based on a blood glucose value of the user within second duration after the third meal time.

In this way, the user can learn about the intake of each meal, and control the intake of each meal, so that the user can develop a regular dietary habit and control a stable fluctuation of blood glucose.

In some implementations, after the electronic device 100 determines the third meal time, the electronic device 100 may further obtain a blood glucose value of the user within third duration after the third meal time, and display a third blood glucose curve, where the third blood glucose curve is determined based on a plurality of blood glucose values of the user within the third duration after the third meal time.

In this way, after the user has the meal, the user can learn about an actual blood glucose status of the user under influence of the dietary event. This helps the user learn about influence of diet on blood glucose more intuitively, so that the user can control blood glucose based on the diet.

In some implementations, the electronic device 100 may measure absorption indexes of a plurality of foods for the user, and store the absorption indexes. Therefore, the electronic device 100 needs to predict a postprandial blood glucose value of the user before the user has the meal. The electronic device 100 may display different foods and absorption indexes of different foods for the user to the user for viewing, and after the user selects a specified food, predict a future blood glucose status of the user based on an absorption index of the food for the user.

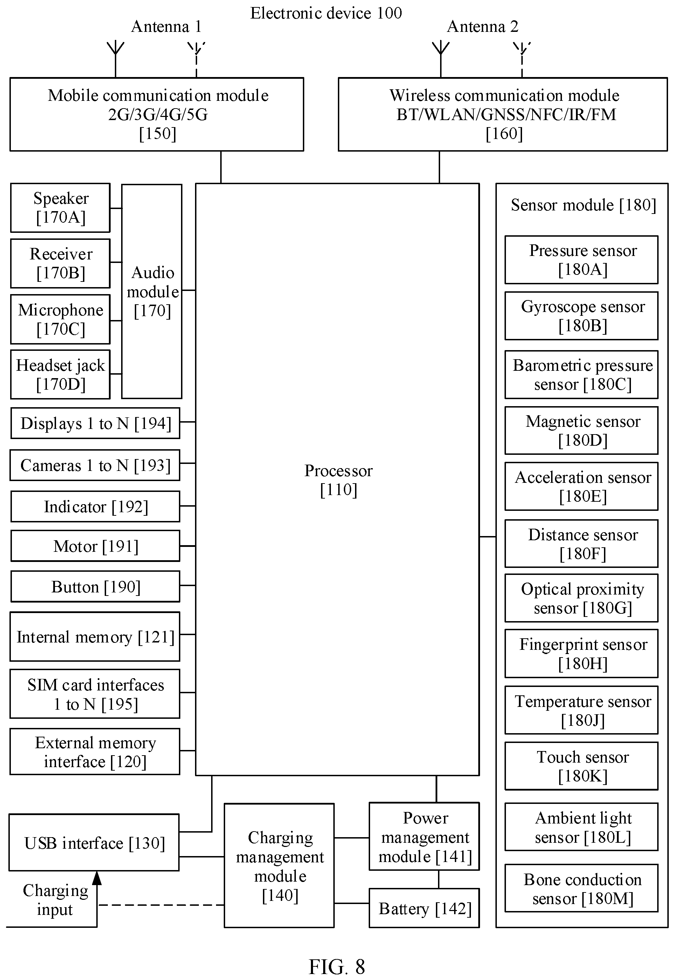

**FIG. 8 is a diagram of a hardware structure of an electronic device 100.**

The electronic device 100 may be a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, an in-vehicle device, a smart home device, and/or a smart city device. A specific type of the electronic device is not particularly limited in embodiments of this application.

The electronic device 100 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identity module (subscriber identity module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

The controller may generate an operation control signal based on an instruction operation code and a time sequence signal, to complete control over instruction fetching and instruction execution.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor 110 may invoke the instructions or the data directly from the memory. This avoids repeated access, reduces waiting time of the processor 110, and improves system efficiency.

**In some implementations, the processor 110 may be configured to determine a historical meal time t1 based on historical blood glucose values of a user, determine a historical meal time t2 based on any one or more of the following: a meal time input by the user, a hand movement of the user, and a heart rate of the user, determine a meal time t0 of the user based on the historical meal time t1 and the historical meal time t2, and evaluate a blood glucose health status or the like of the user based on the meal time t0 of the user. Alternatively, the processor 110**

**may be configured to determine an absorption index of a food for the user based on a blood glucose value of the user in a time period and a physical condition of the user after the user ingests a specific intake of the food.**

A wireless communication function of the electronic device 100 may be implemented by using the antenna 1, the antenna 2, the mobile communication module 150, the wireless communication module 160, the modem processor, the baseband processor, and the like.

The antenna 1 and the antenna 2 are configured to transmit and receive electromagnetic wave signals. Each antenna in the electronic device 100 may be configured to cover one or more communication frequency bands. Different antennas may be further reused to improve antenna utilization. For example, the antenna 1 may be reused as a diversity antenna in a wireless local area network. In some other embodiments, the antenna may be used in combination with a tuning switch.

The mobile communication module 150 may provide a wireless communication solution including 2G/3G/4G/5G or the like and applied to the electronic device 100. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the processed electromagnetic wave to the modem processor for demodulation. The mobile communication module 150 may further amplify a signal modulated by the modem processor, and convert the amplified signal through the antenna 1 into an electromagnetic wave for radiation. In some embodiments, at least some functional modules in the mobile communication module 150 may be disposed in the processor 110. In some embodiments, at least some functional modules in the mobile communication module 150 and at least some modules in the processor 110 may be disposed in a same component.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then the demodulator transmits the low-frequency baseband signal obtained through demodulation to the baseband processor for processing. The low-frequency baseband signal is processed by the baseband processor and then transmitted to the application processor. The application processor outputs a sound signal through an audio device (not limited to the speaker 170A, the receiver 170B, or the like), or displays an image or a video through the display 194. In some embodiments, the modem processor may be an independent component. In some other embodiments, the modem processor may be independent of the processor 110, and is disposed in a same component as the mobile communication module 150 or another functional module.

The wireless communication module 160 may provide a wireless communication solution including a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, and the like and applied to the electronic device 100. The wireless communication module 160 may be one or more components integrating at least one communication processing module. The wireless communication module 160 receives an electromagnetic wave through the antenna 2, performs demodulation and filtering processing on an electromagnetic wave signal, and sends the processed signal to the processor 110. The wireless communication module 160 may further receive a to-be-sent signal from the processor 110, perform frequency modulation and amplification on the signal, and convert the signal through the antenna 2 into an electromagnetic wave for radiation.

In some embodiments, the antenna 1 of the electronic device 100 is coupled to the mobile communication module 150, and the antenna 2 is coupled to the wireless communication module 160, so that the electronic device 100 can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, the GNSS, the WLAN, NFC, FM, the IR technology, and/or the like. The GNSS may include a global satellite positioning system (global positioning system, GPS), a global navigation satellite system (global navigation satellite system, GLONASS), a BeiDou navigation satellite system (BeiDou navigation satellite system, BDS), a quasi-zenith satellite system (quasi-zenith satellite system, QZSS), and/or a satellite based augmentation system (satellite based augmentation system, SBAS).

**In some implementations, the electronic device 100 may establish a communication connection to an electronic device 200 by using the mobile communication module 150 or the wireless communication module 160, and obtain a blood glucose value collected by the electronic device 200.**

The electronic device 100 implements a display function by using the GPU, the display 194, the application processor, and the like. The GPU is a microprocessor for image processing, and is connected to the display 194 and the application processor. The GPU is configured to perform mathematical and geometric computation for graphics rendering.

The processor 110 may include one or more GPUs, and the one or more GPUs execute program instructions to generate or change display information.

The display 194 is configured to display an image, a video, and the like. In some embodiments, the electronic device may include one or N displays 194, where N is a positive integer greater than 1.

**In some implementations, the display 194 may be configured to: when the meal time t0 of the user that is determined by the electronic device 100 is reached, display prompt information to prompt the user that the predicted meal time is reached currently, and display a blood glucose curve predicted by the electronic device 100, a blood glucose curve actually monitored by using the electronic device 200 after the user has a meal, and the like.**

The electronic device 100 may implement a photographing function by using the ISP, the camera 193, the video codec, the GPU, the display 194, the application processor, and the like.

The camera 193 is configured to capture a still image or a video. In some embodiments, the electronic device 100 may include one or N cameras 193, where N is a positive integer greater than 1.

The internal memory 121 may include one or more random access memories (random access memories, RAMs) and one or more non-volatile memories (non-volatile memories, NVMs).

The random access memory may be directly read and written by the processor 110. The random access memory may be configured to store an executable program (for example, machine instructions) in an operating system or another running program, and may be further configured to store data of the user, data of an application, and the like.

The non-volatile memory may also store the executable program, the data of the user, the data of the application, and the like, which may be pre-loaded to the random access memory for the processor 110 to directly perform a read or write operation.

The electronic device 100 may implement an audio function by using the audio module 170, the speaker 170A, the receiver 170B, the microphone 170C, the headset jack 170D, the application processor, or the like, for example, music playback and sound recording.

The speaker 170A, also referred to as a "loudspeaker", is configured to convert an electrical audio signal into a sound signal. The electronic device 100 may be used to listen to music or answer a call in a hands-free mode over the speaker 170A.

The gyroscope sensor 180B may be configured to determine a motion posture of the electronic device 100. In some embodiments, angular velocities of the electronic device 100 around three axes (that is, an x-axis, a y-axis, and a z-axis) may be determined by using the gyroscope sensor 180B. The gyroscope sensor 180B may be configured to implement image stabilization during photographing. For example, when the shutter is pressed, the gyroscope sensor 180B detects an angle at which the electronic device 100 shakes, calculates, based on the angle, a distance for which a lens module needs to compensate, and drives the lens to move in an opposite direction to counteract the shake of the electronic device 100, thereby implementing image stabilization. The gyroscope sensor 180B may also be used in a navigation scenario and a motion-sensing game scenario.

The acceleration sensor 180E may detect magnitudes of accelerations of the electronic device 100 in various directions (generally three axes). When the electronic device 100 is stationary, a magnitude and a direction of gravity may be detected. The acceleration sensor 180E may be further configured to identify a posture of the electronic device, and is used in an application such as switching between a landscape mode and a portrait mode or a pedometer.

**In some implementations, the electronic device 100 may detect a hand movement of the user by using the gyroscope sensor 180B and the acceleration sensor 180E, and determine a meal time of the user when the hand movement of the user meets a preset condition.**

The bone conduction sensor 180M may obtain a vibration signal. In some embodiments, the bone conduction sensor 180M may obtain a vibration signal of a bone segment that vibrates in response to vocalization of a human body. The bone conduction sensor 180M may also be in contact with a human pulse to receive a blood pressure pulsation signal. In some embodiments, the bone conduction sensor 180M may alternatively be disposed in a headset and combined with the headset into a bone conduction headset. The audio module 170 may obtain a voice signal through parsing based on the vibration signal that is of the bone segment vibrating in response to the vocalization and that is obtained by the bone conduction sensor 180M, to implement a voice function. The application processor may parse heart rate information based on the blood pressure pulsation signal obtained by the bone conduction sensor 180M, to implement a heart rate detection function.

**In some implementations, the electronic device 100 may detect a heart rate of the user by using the bone conduction sensor 180M, and determine a meal time of the user when the heart rate of the user meets a preset condition.**

It may be understood that the structure shown in this embodiment of the present invention does not constitute a specific limitation on the electronic device 100. In some other embodiments of this application, the electronic device 100 may include more or fewer components than those shown in the figure, some components may be combined, or some components may be split, or different component arrangements may be used. The components shown in the figure may be implemented by hardware, software, or a combination of software and hardware.

The electronic device may be a portable terminal device running HarmonyOS, iOS, Android, Microsoft, or another operating system, for example, a mobile phone, a tablet computer, or a wearable device; or may be a non-portable terminal device, for example, a laptop (Laptop) computer having a touch-sensitive surface or a touch panel, or a desktop computer having a touch-sensitive surface or a touch panel. A software system of the electronic device 100 may use a layered architecture, an event-driven architecture, a microkernel architecture, a micro service architecture, or a cloud architecture. In this embodiment of the present invention, an Android system with a layered architecture is used as an example to illustrate a software structure of the electronic device 100.

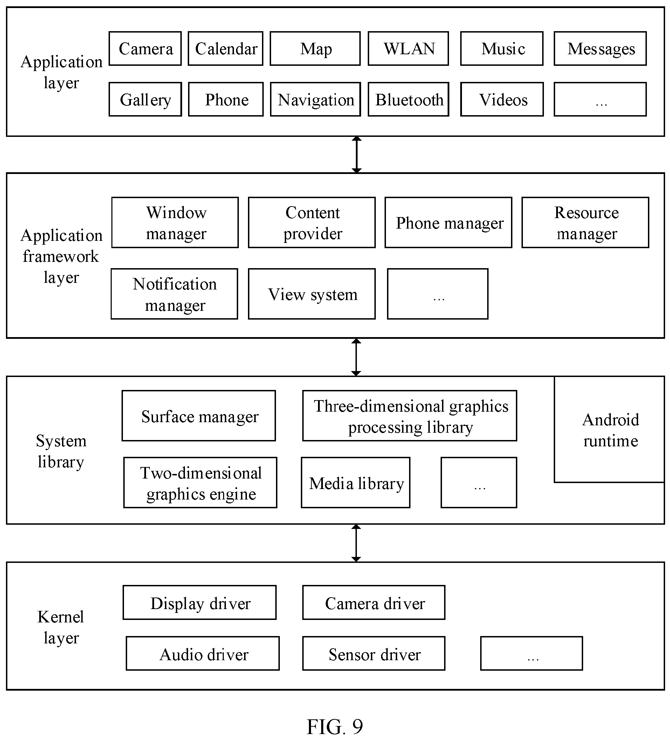

**FIG. 9 is a block diagram of the software structure of the electronic device 100 according to an embodiment of this application.**

In the layered architecture, software is divided into several layers, and each layer has a clear role and task. The layers communicate with each other through a software interface. In some embodiments, the Android system is divided into four layers: an application layer, an application framework layer, an Android runtime (Android runtime) and system library, and a kernel layer from top to bottom.

The application layer may include a series of application packages.

As shown in FIG. 9, the application packages may include applications such as Camera, Gallery, Calendar, Phone, Map, Navigation, WLAN, Bluetooth, Music, Videos, and Messages.

The application framework layer provides an application programming interface (application programming interface, API) and a programming framework for the applications at the application layer. The application framework layer includes some predefined functions.

As shown in FIG. 9, the application framework layer may include a window manager, a content provider, a view system, a phone manager, a resource manager, a notification manager, and the like.

The window manager is configured to manage a window program. The window manager may obtain a size of the display, determine whether there is a status bar, perform screen locking, take a screenshot, and the like.

The content provider is configured to store and obtain data, and make the data accessible to an application. The data may include a video, an image, audio, calls made and answered, a browsing history and bookmarks, a phone book, and the like.

The view system includes visual controls such as a control for displaying a text and a control for displaying an image. The view system may be configured to construct an application. A display interface may include one or more views. For example, a display interface including an SMS message notification icon may include a text display view and an image display view.

The phone manager is configured to provide a communication function for the electronic device 100, for example, call status management (including answering or ending a call, or the like).

The resource manager provides various resources such as a localized character string, an icon, an image, a layout file, and a video file for an application.

The notification manager enables an application to display notification information in the status bar, and may be configured to convey a notification message, which may automatically disappear after a short time without requiring user interaction. For example, the notification manager is configured to notify download completion, give a message notification, and the like. The notification manager may alternatively present a notification in a top status bar of the system in a form of a graph or a scrolling text, for example, a notification of an application running in the background, or may be a notification that appears on the screen in a form of a dialog window. For example, text information is displayed in the status bar, an alert tone is played, the electronic device vibrates, or the indicator blinks.

The Android runtime includes a core library and a virtual machine. The Android runtime is responsible for scheduling and management of the Android system.

The core library includes two parts: a function that needs to be called in Java language and a core library of Android.

The application layer and the application framework layer run on the virtual machine. The virtual machine executes Java files at the application layer and the application framework layer as binary files. The virtual machine is configured to implement functions such as object lifecycle management, stack management, thread management, security and exception management, and garbage collection.

The system library may include a plurality of functional modules, for example, a surface manager (surface manager), a media library (Media Library), a three-dimensional graphics processing library (for example, OpenGL ES), and a 2D graphics engine (for example, SGL).

The surface manager is configured to manage a display subsystem and provide fusion of 2D and 3D layers for a plurality of applications.

The media library supports playback and recording in a plurality of commonly used audio and video formats, still image files, and the like. The media library may support a plurality of audio and video coding formats, for example, MPEG-4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working process of software and hardware of the electronic device 100 with reference to a shooting scene.

When the touch sensor 180K receives a touch operation, a corresponding hardware interrupt is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. An example in which the touch operation is a tap operation, and a control corresponding to the tap operation is a control of a camera application icon is used. The camera application invokes an interface of the application framework layer to start the camera application, then starts the camera driver by invoking the kernel layer, and captures a still image or a video through the camera 193.

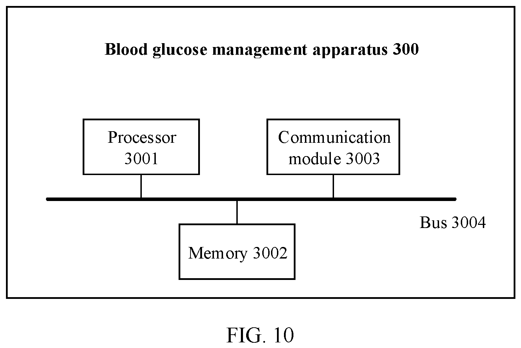

**FIG. 10 is a diagram of a structure of a blood glucose management apparatus 300 according to an embodiment of this application.**

As shown in FIG. 10, the blood glucose management apparatus 300 may be the electronic device 100 in the foregoing embodiment. The blood glucose management apparatus 300 may include components such as a processor 3001, a memory 3002, and a communication module 3003. These components may be connected by using a bus 3004 or in another manner. In FIG. 10, connection by using a bus is used as an example. The bus 3004 is configured to implement connection and communication between the processor 3001, the memory 3002, and the communication module 3003.

The processor 3001 may include one or more processing units. The processor 3001 may be configured to provide computing and control capabilities, to support operating of the entire blood glucose management apparatus 300.

The memory 3002 may be configured to store various software programs and/or a plurality of groups of instructions. Specifically, the memory 3002 may include a high-speed random access memory, and may also include a non-volatile memory, for example, one or more disk storage devices, a flash memory device, or another non-volatile solid-state storage device.

The communication module 3003 may be configured to implement communication between the blood glucose management apparatus 300 and another communication device. Specifically, the communication module 3003 may include a communication interface. The communication interface may be a 3G communication interface, a long term evolution (LTE) (4G) communication interface, a 5G communication interface, a WLAN communication interface, a WAN communication interface, a human-body surface communication interface, or the like. In addition to a wireless communication interface, the blood glucose management apparatus 300 may be further configured with a wired communication interface to support wired communication.

In some implementations, the processor 3001 may be configured to determine a historical meal time t1 based on historical blood glucose values of a user, determine a historical meal time t2 based on any one or more of the following: a meal time input by the user, a hand movement of the user, and a heart rate of the user, determine a meal time t0 of the user based on the historical meal time t1 and the historical meal time t2, and evaluate a blood glucose health status of the user based on the meal time t0 of the user.

In some other implementations, the processor 3001 may be configured to determine an absorption index of a food for the user based on a blood glucose value of the user in a time period and a physical condition of the user after the user ingests a specific intake of the food.

In some implementations, the memory 3002 may be configured to store software or program code required for all or some functions of the electronic device 100 in the foregoing method embodiments. In addition, the memory 3002 may be configured to store the historical meal time t1, the historical meal time t2, and the meal time t0 of the user, or the memory 3002 may be configured to store an absorption index of one or more foods for the user.

In some implementations, the communication module 3003 may be configured to communicate with an electronic device 200, to obtain a blood glucose value collected by the electronic device 200.

For specific operations and beneficial effects of the units in the blood glucose management apparatus 300 shown in FIG. 10, refer to corresponding descriptions in the method embodiments. Details are not described herein again.

It may be understood that the embodiment shown in FIG. 10 is merely an example. In this embodiment of this application, the blood glucose management apparatus 300 may further include more or fewer components than those in the embodiment shown in FIG. 10, or components different from those in the embodiment shown in FIG. 10. This is not limited in this application. It should be understood that the steps in the foregoing method embodiments may be completed by using an integrated logic circuit of hardware in the processor or instructions in a form of software. The steps of the methods disclosed with reference to embodiments of this application may be directly performed and completed by a hardware processor, or may be performed and completed by using a combination of hardware in the processor and a software module.

This application further provides an electronic device, including a memory and a processor. The memory may be configured to store a computer program. The processor may be configured to invoke the computer program in the memory, so that the electronic device performs the method performed by the electronic device 100 in any one of the foregoing embodiments.

This application further provides a chip system. The chip system includes at least one processor, configured to implement functions in the method performed by the electronic device 100 in any one of the foregoing embodiments.

In a possible design, the chip system further includes a memory, the memory is configured to store program instructions and data, and the memory is located inside or outside the processor.

The chip system may include a chip, or may include a chip and another discrete component.

Optionally, there may be one or more processors in the chip system. The processor may be implemented by using hardware, or may be implemented by using software. When the processor is implemented by using the hardware, the processor may be a logic circuit, an integrated circuit, or the like. When the processor is implemented by using the software, the processor may be a general-purpose processor, and is implemented by reading software code stored in the memory.

Optionally, there may also be one or more memories in the chip system. The memory may be integrated with the processor, or may be disposed separately from the processor. This is not limited in embodiments of this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in embodiments of this application.

For example, the chip system may be a field programmable gate array (field programmable gate array, FPGA), an application-specific integrated circuit (application-specific integrated circuit, ASIC), a system on chip (system on chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

This application further provides a computer program product. The computer program product includes a computer program (which may also be referred to as code or instructions). When the computer program is run, a computer is enabled to perform the method performed by the electronic device 100 in any one of the foregoing embodiments.

This application further provides a computer-readable storage medium. The computer-readable storage medium stores a computer program (which may also be referred to as code or instructions). When the computer program is run, a computer is enabled to perform the method performed by the electronic device 100 in any one of the foregoing embodiments.

It should be noted that, the processor in embodiments of this application may be an integrated circuit chip, and has a signal processing capability. In an implementation process, the steps of the foregoing method embodiments may be completed by a hardware integrated logic circuit in the processor or by using instructions in a form of software. The processor may be a general-purpose processor, a digital signal processor (digital signal processor, DSP), an application-specific integrated circuit (application specific integrated circuit, ASIC), a field programmable gate array (field programmable gate array, FPGA) or another programmable logic device, a discrete gate or transistor logic device, or a discrete hardware component. The processor may implement or perform the methods, steps, and logical block diagrams that are disclosed in embodiments of this application. The general-purpose processor may be a microprocessor, or the processor may be any conventional processor or the like. The steps in the methods disclosed with reference to embodiments of this application may be directly performed and completed by a hardware decoding processor, or may be performed and completed by using a combination of hardware in the decoding processor and a software module. The software module may be located in a mature storage medium in the art, such as a random access memory, a flash memory, a read-only memory, a programmable read-only memory, an electrically erasable programmable memory, or a register. The storage medium is located in the memory. The processor reads information in the memory and completes the steps of the foregoing methods in combination with hardware of the processor.

In addition, an embodiment of this application further provides an apparatus. The apparatus may be specifically a component or a module, and the apparatus may include one or more processors and memories that are connected to each other. The memory is configured to store a computer program. When the computer program is executed by the one or more processors, the apparatus is enabled to perform the methods in the foregoing method embodiments.

The apparatus, the computer-readable storage medium, the computer program product, or the chip provided in embodiments of this application is configured to perform the corresponding method provided above. Therefore, for beneficial effects that can be achieved, refer to beneficial effects in the corresponding method provided above, and details are not described herein again.

Implementations of this application may be randomly combined to achieve different technical effects.

All or some of the foregoing embodiments may be implemented by software, hardware, firmware, or any combination thereof. When software is used to implement the embodiments, all or some of the embodiments may be implemented in a form of a computer program product. The computer program product includes one or more computer instructions. When the computer program instructions are loaded and executed on the computer, the procedure or functions according to this application are all or partially generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid state disk (solid state disk, SSD)), or the like.

A person of ordinary skill in the art may understand that all or some of the procedures of the methods in the foregoing embodiments may be implemented by a computer program instructing relevant hardware. The program may be stored in a computer-readable storage medium. When the program is executed, the procedures of the methods in the foregoing embodiments may be performed. The storage medium includes any medium that can store program code, such as a ROM, a random access memory RAM, a magnetic disk, or an optical disc.

In summary, the foregoing descriptions are merely embodiments of the technical solutions of the present invention, but are not intended to limit the protection scope of the present invention. Any modification, equivalent replacement, improvement, and the like based on the disclosure of the present invention shall fall within the protection scope of the present invention.

**Claims**

1. A blood glucose management method, wherein the method is applied to an electronic device, and the method comprises:

   determining a first meal time based on historical blood glucose values of a user;
   determining a second meal time based on any one or more of the following: a meal time input by the user, a hand movement of the user, and a heart rate of the user; and
   determining a third meal time based on the first meal time and the second meal time, wherein the third meal time is used by the electronic device to evaluate a blood glucose status of the user.

2. The method according to claim 1, wherein after the electronic device determines the third meal time based on the first meal time and the second meal time, the method further comprises:
   outputting first prompt information when the third meal time is reached, wherein the first prompt information indicates that a current time reaches a meal time predicted by the electronic device.

3. The method according to claim 2, wherein the method further comprises:
   detecting a first operation performed on the first prompt information, and determining the third meal time as a meal time of a first dietary event of the user, or determining a fourth meal time as a meal time of a first dietary event of the user, wherein the fourth meal time is different from the third meal time.

4. The method according to claim 2, wherein the fourth meal time is a time input by the user or a time determined based on the third meal time.

5. The method according to claim 2, wherein after determining the fourth meal time as the meal time of the first dietary event of the user and detecting the first operation performed on the first prompt information, the method further comprises:

   displaying the first prompt information after a first time interval; and
   detecting a second operation performed on the first prompt information, and determining that the current time as the fourth meal time.

6. The method according to any one of claims 3 to 5, wherein before or after determining the meal time of the first dietary event, the method further comprises:

   determining a first food to be ingested by the user and a first intake of the first food in the first dietary event;

determining a first blood glucose curve based on information about the first food, the first intake of the first food, and a blood glucose value of the user before a current time point, wherein the first blood glucose curve is a blood glucose change of the user, predicted by the electronic device, under influence of the first dietary event; and displaying the first blood glucose curve.

7. The method according to claim 6, wherein the method further comprises:

determining a second blood glucose curve based on the blood glucose value of the user before the current time point, wherein the second blood glucose curve is a blood glucose change of the user, predicted by the electronic device, in absence of the first dietary event; and displaying the second blood glucose curve.

8. The method according to claim 6 or 7, wherein determining the first blood glucose curve based on the information about the first food, the first intake of the first food, and the blood glucose value of the user before the current time point specifically comprises: determining the first blood glucose curve based on an absorption index of the first food for the user, the first intake of the first food, and the blood glucose value of the user before the current time point, wherein the absorption index of the first food for the user is related to the first food and a physical condition of the user.

9. The method according to claim 8, wherein the method further comprises: determining the absorption index of the first food for the user based on a blood glucose value within first duration and the physical condition after the user ingests a second intake of the first food, wherein the physical condition comprises any one or more of the following: an age, a height, a weight, a disease type, and medication information.

10. The method according to any one of claims 1 to 9, wherein the electronic device stores absorption indexes of a plurality of foods for the user.

11. The method according to any one of claims 1 to 10, wherein the method further comprises:

determining a third intake of the user based on a blood glucose value of the user within second duration after the third meal time; and displaying the third intake.

12. The method according to any one of claims 1 to 11, wherein the method further comprises:

obtaining a blood glucose value of the user within third duration after the third meal time; and displaying a third blood glucose curve, wherein the third blood glucose curve is determined based on a plurality of blood glucose values of the user within the third duration after the third meal time.

13. The method according to any one of claims 1 to 12, wherein determining the first meal time based on the historical blood glucose values of the user specifically comprises: determining the first meal time based on a rate of increase in the historical blood glucose values of the user.

14. An electronic device, comprising a memory, one or more processors, and one or more programs, wherein when the one or more processors execute the one or more programs, the electronic device is enabled to implement the method according to any one of claims 1 to 13.

15. A computer-readable storage medium, comprising instructions, wherein when the instructions are run on an electronic device, the electronic device is enabled to perform the method according to any one of claims 1 to 13.

**Communication system 1000**

Electronic
device 100

Electronic
device 200

FIG. 1

| Predict a meal time of a user | S101 |

| Calibrate the meal time | S102 |

| Evaluate a blood glucose status of the user based on the meal time | S103 |

| Output a suggestion on blood glucose of the user | S104 |

FIG. 2

**Daily Dietary Analysis**  📅 August 10

Page A

Carbohydrate intake analysis

A1

| 120 g | | 32 g | 52 g | | 36 g |
|---|---|---|---|---|---|

Total carbohydrate intake      Breakfast   Lunch        Dinner

Today's intake  ⬭  120 g

Yesterday's intake  ⬭  140 g

Breakfast                                    Modify meal time ›

🥣 7:25 to 7:40    —— Today's data    ········· Yesterday's data

6:00          9:00          11:00

A2

Today's blood glucose is expected to increase by 2.3 mmol/L compared with yesterday, the carbohydrate intake in the morning has increased by 6 g compared with yesterday, and balanced diet and balanced carbohydrate are suggested

A3

Estimated carbohydrate absorption amount (g/0.5 hour)    15 g

Suitable
▼

0      5      10          20          30

A4

FIG. 3A

**Dietary Analysis Report** — Page B

August 1 (Past 10 days) August 10

Breakfast — B1

30 g

Average

**Fast increase and fast decrease**
Blood glucose mode

Postprandial blood glucose graph — B2

6:00    9:00    11:00

Breakfast carbohydrate intake graph — B3

8/1 8/2 8/3 8/4 8/5 8/6 8/7 8/8 8/9 8/10

B4 — Dietary event recording — B4-1

Algorithm automatic recording — B4-2

Breakfast (10 meals) — B5

1. Preprandial blood glucose     3.10 ✓

2. Postprandial peak blood glucose   [Slightly high] 10.31 ⬆

3. Time to reach peak blood glucose  7:30

4. Postprandial blood glucose duration   1:30

5. 2-hour postprandial blood glucose   3.24

FIG. 3B

Determine a historical meal time t1 of a user based on historical blood glucose values of the user ∼ S201

Determine a historical meal time t2 based on any one or more of the following: a historical meal time input by the user, a hand movement of the user, and a heart rate of the user ∼ S202

Determine a meal time t0 of the user based on the historical meal time t1 and the historical meal time t2 ∼ S203

When the meal time t0 of the user is reached, output prompt information, where the prompt information is used to prompt the user that a predicted meal time has been reached currently ∼ S204

FIG. 4

User interface 10

FIG. 5A

User interface 10

FIG. 5B

User interface 10

FIG. 5C

User interface 10

The following shows a blood glucose fluctuation predicted for this meal:

104

Start the meal

104A

6:00    7:25    9:00

—— Blood glucose curve predicted in presence of a dietary event

······ Blood glucose curve predicted in absence of a dietary event

It is predicted that your meal may cause blood glucose to rapidly increase and exceed a normal range. Please pay attention to glucose control.

104B

Previous    Confirm

104C    104D

07:25

August 10, Friday

30°C
Beijing

08:08

FIG. 5D

User interface 20

4G 4G 📶      🔋 08:08

Start now 〉   201

**Absorption index measurement**

FIG. 6A

User interface 30

301

Cancel                                              Confirm

302

08:08

**Step 1: Keep fasting before measurement**

Input the following information:

Age _____

Height _____

Weight _____

Disease type _____

Medication
information _____

303

FIG. 6B

User interface 40

Cancel     Start countdown

401

402

**Step 2: Prepare a food whose absorption index needs to be measured**

Input the food whose absorption index is to be measured: _____

403

FIG. 6C

User interface 50

501

Cancel    Complete    502

**Step 3: Within the countdown time, finish eating the food whose absorption index needs to be measured**

5:00:00    503

Precaution: Do not ingest other foods or exercise strenuously within the countdown time

FIG. 6D

**User interface 60**

4G.ill 4G.ill 📶　　　　　　🔋 08:08

Confirm 〉　　⌒ 601

Step 4: The background is continuously
monitoring your blood glucose status.
Please view the absorption index
measurement result after two hours　　⌒ 602

FIG. 6E

User interface 70

| Food | Absorption index |
|:----:|:----------------:|
| XXX | XXX |

Absorption index measurement result

701

FIG. 6F

Obtain a blood glucose value within first duration after a user inputs a second intake of a first food — S301

Determine an absorption index of the first food for the user based on the blood glucose value within the first duration and a physical condition of the user — S302

FIG. 7

Electronic device 100

Antenna 1

Antenna 2

| Mobile communication module 2G/3G/4G/5G [150] | Wireless communication module BT/WLAN/GNSS/NFC/IR/FM [160] |

Speaker [170A]

Receiver [170B]

Audio module [170]

Microphone [170C]

Headset jack [170D]

Displays 1 to N [194]

Cameras 1 to N [193]

Indicator [192]

Motor [191]

Button [190]

Internal memory [121]

SIM card interfaces 1 to N [195]

External memory interface [120]

Processor [110]

Sensor module [180]

Pressure sensor [180A]

Gyroscope sensor [180B]

Barometric pressure sensor [180C]

Magnetic sensor [180D]

Acceleration sensor [180E]

Distance sensor [180F]

Optical proximity sensor [180G]

Fingerprint sensor [180H]

Temperature sensor [180J]

Touch sensor [180K]

Ambient light sensor [180L]

Bone conduction sensor [180M]

USB interface [130]

Charging input

Charging management module [140]

Power management module [141]

Battery [142]

FIG. 8

| Application layer | Camera | Calendar | Map | WLAN | Music | Messages |
|---|---|---|---|---|---|---|
| | Gallery | Phone | Navigation | Bluetooth | Videos | ... |

| Application framework layer | Window manager | Content provider | Phone manager | Resource manager |
|---|---|---|---|---|
| | Notification manager | View system | ... | |

| System library | Surface manager | Three-dimensional graphics processing library | Android runtime |
|---|---|---|---|
| | Two-dimensional graphics engine | Media library | ... | |

| Kernel layer | Display driver | Camera driver |
|---|---|---|
| | Audio driver | Sensor driver | ... |

FIG. 9

Blood glucose management apparatus 300

| Processor 3001 | | Communication module 3003 |

Bus 3004

Memory 3002

FIG. 10

## INTERNATIONAL SEARCH REPORT

International application No.

**PCT/CN2024/143474**

### A. CLASSIFICATION OF SUBJECT MATTER

G16H 50/30(2018.01)i; G16H20/60(2018.01)i; A61B5/145(2006.01)i; A61B5/024(2006.01)i; A61B5/00(2006.01)i

According to International Patent Classification (IPC) or to both national classification and IPC

### B. FIELDS SEARCHED

Minimum documentation searched (classification system followed by classification symbols)

IPC: G16H: A61B

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VCN, VEN, CJFD, CNKI, ISI-WEB OF SCIENCE, PUBMED, BING, STNext, 百度, BAIDU, 百度学术, BAIDU SCHOLAR, 读秀, DUXIU: 华为, 田侃, 潘骏, 解松林, 张杰, 血糖, 血糖曲线, 上升速率, 升高, 变化, 进餐, 就餐, 吃饭, 饮食, 时间, 时刻, 提示, 提醒, 摄入量, 摄取, 吸收指数, 吸收, 代谢, 身体, 年龄, 体重, 身高, 病, 药, HUAWEI, blood glucose, blood glucose profile, rate of rise, rise, change, meal, eat, diet, time, moment, cue, reminder, intake, absorption index, absorpt, metabolism, body, age, weight, height, ill, disease, medication

### C. DOCUMENTS CONSIDERED TO BE RELEVANT

| Category* | Citation of document, with indication, where appropriate, of the relevant passages | Relevant to claim No. |
|---|---|---|
| PY | CN 118298992 A (HUAWEI TECHNOLOGIES CO., LTD.) 05 July 2024 (2024-07-05) claims 1-3, 5, 6, 8, 11-13, and 25-34, and description, paragraphs [0032], [0124], [0187], [0197]-[0203], [0239]-[0256], [0261]-[0264], and [0568] | 1-15 |
| PY | CN 118315007 A (DACHUAN DISTRICT PEOPLE'S HOSPITAL OF DAZHOU CITY) 09 July 2024 (2024-07-09) claim 1 | 2-15 |
| Y | CN 108348198 A (SAMSUNG ELECTRONICS CO., LTD.) 31 July 2018 (2018-07-31) claims 1, 2, 4, 5, 12-14, 16-18, and 20, and description, paragraphs [0006], [0078]-[0081], [0083]-[0088], [0096]-[0102], [0105]-[0108], [0110], [0131]-[0153], and [0194]-[0198] | 1-15 |
| Y | CN 112687372 A (ZHUHAI MEIZU TECHNOLOGY CO., LTD.) 20 April 2021 (2021-04-20) claims 1 and 2, and description, paragraphs [0038]-[0046] and [0068] | 1-15 |

☑ Further documents are listed in the continuation of Box C.　　☑ See patent family annex.

| | |
|---|---|
| *　Special categories of cited documents: | "T"　later document published after the international filing date or priority date and not in conflict with the application but cited to understand the principle or theory underlying the invention |
| "A"　document defining the general state of the art which is not considered to be of particular relevance | |
| "D"　document cited by the applicant in the international application | "X"　document of particular relevance; the claimed invention cannot be considered novel or cannot be considered to involve an inventive step when the document is taken alone |
| "E"　earlier application or patent but published on or after the international filing date | |
| "L"　document which may throw doubts on priority claim(s) or which is cited to establish the publication date of another citation or other special reason (as specified) | "Y"　document of particular relevance; the claimed invention cannot be considered to involve an inventive step when the document is combined with one or more other such documents, such combination being obvious to a person skilled in the art |
| "O"　document referring to an oral disclosure, use, exhibition or other means | "&"　document member of the same patent family |
| "P"　document published prior to the international filing date but later than the priority date claimed | |

| Date of the actual completion of the international search | Date of mailing of the international search report |
|---|---|
| **26 March 2025** | **01 April 2025** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2024/143474** |

### C. DOCUMENTS CONSIDERED TO BE RELEVANT

| Category* | Citation of document, with indication, where appropriate, of the relevant passages | Relevant to claim No. |
| --- | --- | --- |
| Y | CN 111508582 A (3722 (BEIJING) HEALTH CONSULTING CO., LTD.) 07 August 2020 (2020-08-07) claims 1-6, 9, and 10, and description, paragraphs [0026]-[0028] | 6-15 |
| Y | CN 114159052 A (WANG ZHIXUAN) 11 March 2022 (2022-03-11) claims 1-4, and description, paragraphs [0015]-[0016] and [0018] | 6-15 |
| Y | CN 107924548 A (GOOGLE INC.) 17 April 2018 (2018-04-17) claims 10-16 and 18-19, and description, paragraph [0028] | 1-15 |
| Y | CN 112509670 A (CHONGQING COLLEGE OF ELECTRONIC ENGINEERING) 16 March 2021 (2021-03-16) claims 1-3 and 5 | 1-15 |
| Y | CN 113080949 A (BEIJING JINGDONG TUOXIAN TECHNOLOGY CO., LTD.) 09 July 2021 (2021-07-09) claims 1-15, and description, paragraphs [0050]-[0061] | 1-15 |

Form PCT/ISA/210 (second sheet) (July 2022)

**INTERNATIONAL SEARCH REPORT**
Information on patent family members

International application No.

**PCT/CN2024/143474**

| Patent document cited in search report | | | Publication date (day/month/year) | Patent family member(s) | | | Publication date (day/month/year) |
|---|---|---|---|---|---|---|---|
| CN | 118298992 | A | 05 July 2024 | None | | | |
| CN | 118315007 | A | 09 July 2024 | None | | | |
| CN | 108348198 | A | 31 July 2018 | KR | 20170055409 | A | 19 May 2017 |
| | | | | KR | 102564489 | B1 | 07 August 2023 |
| | | | | EP | 3363361 | A4 | 22 August 2018 |
| | | | | EP | 3363361 | A1 | 11 November 2020 |
| | | | | US | 2018344259 | A1 | 06 December 2018 |
| | | | | US | 11103195 | B2 | 31 August 2021 |
| | | | | RU | 2015148522 | A | 19 May 2017 |
| | | | | RU | 2626672 | C2 | 31 July 2017 |
| | | | | WO | 2017082525 | A1 | 18 May 2017 |
| CN | 112687372 | A | 20 April 2021 | None | | | |
| CN | 111508582 | A | 07 August 2020 | None | | | |
| CN | 114159052 | A | 11 March 2022 | None | | | |
| CN | 107924548 | A | 17 April 2018 | EP | 3262598 | A1 | 03 January 2018 |
| | | | | EP | 3262598 | A4 | 22 August 2018 |
| | | | | WO | 2017027258 | A1 | 16 February 2017 |
| | | | | US | 2017046800 | A1 | 16 February 2017 |
| | | | | US | 10482551 | B2 | 19 November 2019 |
| | | | | DE | 202016007875 | U1 | 21 February 2017 |
| CN | 112509670 | A | 16 March 2021 | None | | | |
| CN | 113080949 | A | 09 July 2021 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311870197X